# EUROPEAN PATENT SPECIFICATION

(11) **EP 3 658 679 B1**
(45) Date of publication and mention of the grant of the patent: **06.09.2023**
(21) Application number: 18837909.3
(22) Date of filing: 23.07.2018
(51) Int. Cl.: C12Q 1/04, G01N 33/18, C12Q 1/70

(54) **METHOD FOR RAPID DETECTION AND ENUMERATION OF VIRUSES, BACTERIOPHAGE AND/OR BACTERIA**
VERFAHREN ZUM SCHNELLEN NACHWEIS UND ZUR AUSZÄHLUNG VON VIREN, BAKTERIOPHAGEN UND/ODER BAKTERIEN
PROCÉDÉ DE DÉTECTION ET D'ÉNUMÉRATION RAPIDES DE VIRUS, DE BACTÉRIOPHAGES ET/OU DE BACTÉRIES

(30) Priority: 23.07.2017 AU 2017902873; 19.11.2017 AU 2017904674; 18.04.2018 AU 2018901288
(43) Date of publication of application: 03.06.2020
(73) Proprietor: Future Biosolutions Pty Ltd, Maroochydore, Queensland 4558 (AU)
(72) Inventor: RAMES, Emily, Maroochydore, Queensland 4558 (AU)
(74) Representative: V.O.
(86) International application number: PCT/AU2018/050765
(87) International publication number: WO 2019/018886

(56) References cited:
- CN-A- 101 650 365
- CN-A- 103 616 512
- US-A- 5 527 667
- MENDEZ J ET AL: "Conservation of phage reference materials and water samples containing bacteriophages of enteric bacteria", JOURNAL OF VIROLOGICAL METHODS, vol. 106, no. 2, 1 December 2002 (2002-12-01), pages 215-224, XP055772584, NL ISSN: 0166-0934, DOI: 10.1016/S0166-0934(02)00163-5
- DERDA RATMIR ET AL: "Filter-Based Assay for Escherichia coli in Aqueous Samples Using Bacteriophage-Based Amplification", ANALYTICAL CHEMISTRY, vol. 85, no. 15, 12 July 2013 (2013-07-12) , pages 7213-7220, XP055772597, ISSN: 0003-2700, DOI: 10.1021/ac400961b Retrieved from the Internet: URL:https://pubs.acs.org/doi/pdf/10.1021/a c400961b>
- MINIKH O ET AL: "Bacteriophage-based biosorbents coupled with bioluminescent ATP assay for rapid concentration and detection of Escherichia coli", JOURNAL OF MICROBIOLOGICAL METHODS, ELSEVIER, AMSTERDAM, NL, vol. 82, no. 2, 1 August 2010 (2010-08-01) , pages 177-183, XP027111464, ISSN: 0167-7012 [retrieved on 2010-06-01]

## Description

### TECHNICAL FIELD

THIS INVENTION relates to a method and/or use of a kit that enables the rapid quantitative enumeration of bacteriophage

### BACKGROUND

Human viruses that can persist in environmental matrices (e.g. water) are associated with diseases such as gastroenteritis, meningitis, myocarditis, hepatitis and respiratory and eye infections. Such human viruses can be present at high numbers in municipal wastewater. Detection of human viruses in water typically indicates faecal or wastewater contamination. Due to the high cost of testing human viruses using culture methods and inadequacy of bacterial indicators for predicting viral contamination, bacteriophages are commonly used as an indicator of faecal viruses/faecal contamination/microbial quality.

Specific bacteriophage types infect and replicate in specific host bacteria types. Thus, a specific host cell bacteria type can be used to detect a specific bacteriophage type. Coliphages, which infect *E*. *coli* (and certain other *Enterobacteriaceae*), are bacteriophage types commonly used as an indicator (or surrogate) of faecal viruses. The two types of coliphages, somatic and F+ coliphages, infect the bacterial host via different routes. Somatic coliphages infect the bacterial host cell via receptors on the outer cell membrane or wall, while F+ coliphages infect bacteria via attachment to the F+ pilus of male strains. F+ coliphages may be further classified as F+ RNA and F+ DNA coliphages, which have RNA and DNA genomes respectively, while somatic coliphages typically have a DNA genome. Each coliphage type has different advantages and limitations for use as an indicator. Coliphages are frequently measured to assess viral disinfection efficiency of treatment processes and/or microbial quality of wastewater/recycled water and drinking water/groundwater. Coliphage monitoring is increasingly advocated in regulatory guidelines for recycled water (e.g. Water Quality Guidelines for Recycled Water Schemes, Queensland Australia, 2008; QLD Public Health Act: Public Health Regulation 2005, Queensland, Australia; Guidelines for validating treatment processes for pathogen reduction, Victoria, Australia 2013) and where treated wastewater augments drinking water supplies (QLD Public Health Act: Public Health Regulation 2005, Queensland, Australia). Guideline levels have recently been set for monitoring coliphages in drinking water in Australia (Australian Drinking Water Guidelines 6, Version 3.2 Updated March 2016) and somatic coliphage monitoring in drinking water in the European Union (EU) has been legislated (http://ec.europa.eu/environment/water/water-drink/review en.html; https://eur-lex.europa.eu/resource.html?uri=cellar:8c5065b2-074f-11e8-b8f5- 01aa75ed71a1.0016.02/DOC_1&format=PDF) in accordance with recommendations of the World Health Organisation (EU) (http://ec.europa.eu/environment/water/water- drink/pdf/WHO_parameter_report.pdf).

Guidelines that may advocate coliphage monitoring for assessing the microbiological safety of recreational water are under development/investigation in the USA (US Environmental Protection Agency, Coliphage Recreational Water Quality Criteria). Coliphages can also be used as an indicator in other applications, such as monitoring combined sewer overflows in high rainfall events, irrigation water quality and food safety.

Coliphages/bacteriophages are also used for various other purposes, such as laboratory methods for determining efficiency of water concentration/recovery methods or assessing viral inactivation potential of treatments (such as UV disinfection, chlorine treatment). A further suite of laboratory methods uses bacteriophages, such as phage display. Further uses of bacteriophages include clinical and industrial/agricultural applications (where bacteriophages are used to treat bacterial infections or otherwise kill target bacteria). A specific bacteriophage type can also be used to detect a specific bacteria type and associated bacterial antibiotic resistance. Specific detection of target bacteria is also relevant for all aforementioned applications (e.g. environmental monitoring, food/beverage safety and clinical applications).

Current culture methods for detecting bacteriophages involve means for detecting the associated lysis of the bacterial host cell resulting from coliphage infection and replication. The conventional method for detecting bacteriophages is the plaque assay. The plaque assay is a cumbersome procedure requiring the tempering of agar and lengthy incubations (~48 hours), and is described in several standard methods (ISO 10705, US-EPA Method 1602: Male-specific (F+) and somatic coliphage in water by single agar layer (SAL) procedure and American Public Health Association: Standard methods for examination of water and wastewater). In the plaque assay, a host bacterial culture and sample are mixed with culture media that contains a low concentration of agar (soft agar). The growth of bacterial host cells throughout the agar layer produces a turbid "lawn" of bacteria. Coliphages cause localised infection of the bacterial host cells (with diffusion being restricted by the gel), resulting in the production of plaques, which are cleared zones of (lysed/dead) bacterial cells (when low numbers of coliphages are present). When high numbers of coliphages are present, the clearing (lysis/death) of bacterial cells is observed throughout the entire bacterial lawn.

Rapid tests for coliphages and other bacteriophages have advantages compared to the plaque assay. Rapid tests for coliphages can enable water treatment processes to be monitored more actively, so that results are provided more timely to water treatment/use, supporting more effective water management for better protection of public health. Simple and inexpensive methods for measuring coliphages/bacteriophages are of value to laboratories which perform large scale/volume testing, and may promote more extensive inclusion of coliphage/viral monitoring in regulatory guidelines for ensuring microbiological water safety. Previously described rapid tests for bacteriophages/coliphages are typically liquid-based assays, for detecting enzymes released from the *E. coli* host cell (due to bacteriophage induced lysis), using fluorescent or chromogenic substrates (US patent US5527667 A, 1996). Various different formats of the test have subsequently been described. A version described by Salter and Durbin (US Patent WO2008143722 A2, 2008; US9376704, 2009), marketed as Charm Fast Phage^{®} (Charm Sciences Inc.), is a liquid assay test kit for detecting coliphages and may also include detection of coliphages within a gel or by continuous flow. The assay was granted US-EPA approval for presence/absence detection of coliphages in groundwater (Salter, R. et al. 2010, Appl Env Microbial 76, 7803-7810). However, lengthy incubation times are still required for this assay (~8 hours), along with overnight incubation for result confirmation. The assay also has disadvantages of low sensitivity or high levels of false positives (i.e. background), as reported by the authors.

Unacceptable background in rapid bacteriophage assays has been proposed to result from the enzyme substrate producing a detectable signal within intact bacterial host cells (not lysed by bacteriophages) and/or due to the requirement for lengthy incubations (~8 hours) wherein higher rates of non-specific bacterial lysis may occur. Various methods for reducing background in rapid bacteriophage assays have been disclosed previously, such as immobilisation of the enzyme substrate or the use of genetically modified organisms/bacteria (GMO). For example, it is stated in WO2001079528A "the use of traditional soluble ES (enzyme substrate) indicators is generally not possible in such assay methods. The soluble ES would undesirably react with enzyme within the intact bacteria cells of both non-target bacteria and, if used, bacterial helper cells and thereby produce unacceptable levels of background signal". WO2001079528A discloses the use of enzyme substrates that are bonded to an insoluble or solid support (immobilised) to thus solve background problems of prior art. The method disclosed by Muniesa and others (US patent WO2015071315 A1, 2015) is a liquid-based assay using GMO or selection of bacterial host strains that are not able to secrete a specified enzyme or uptake the said enzyme substrate, to reduce background signals/false positives; this assay required up to 4.5 hours for detecting low numbers of somatic coliphages, 2-18 h for activation of the bacterial strain and overnight confirmation of results. This method did not achieve low sensitivity (with a sensitivity of ~50 plaque forming units (PFU) per100mL) and has not been successful with F+ coliphages (as disclosed by the author, 2017 UNC Water Microbiology Conference). The method disclosed by Campbell and Trokhina (US patent US20160131591 A1, 2016) also includes use of GMO (bacterial strains which over-express LacZQ or endolysin terminator sequence); and includes description of a particular microfluidic device (a device with network/channels in the micron range) for bacteriophage detection; no claims for quantitation, rapidity or sensitivity were presented. The present invention disclosed herein is not a liquid-based assay, does not use specific bacterial strain selection or genetic modification, microfluidic devices, nor enzyme substrate immobilisation. There are distinct advantages of not using GMO for bacteriophage detection, since use of GMO limits strain availability and use is generally limited to certified facilities such as Physical Containment Level 2 (PC2) in Australia. Non-GMO can be used in any laboratory, including low resource site laboratories and in the field.

Thus, in view of the above information, no simple method for rapid quantitative enumeration of bacteriophages (including low numbers such as1 PFU/100 mL and detection of both somatic and F+ coliphages) within 1.5-4.5 hours and not requiring overnight confirmation, and without the use of selected or GMO strains, enzyme immobilisation or specific microfluidic devices has previously been demonstrated, for analysis of sample volumes relevant for water quality and food analysis (e.g. 1mL to 100 mL) or for analysis of clinical or laboratory samples/isolates; nor has a rapid method been disclosed that provides directly equivalent results to the plaque assay. Assays with these characteristics are highly demanded for rapid detection of bacteriophages (and bacteria/anti-biotic resistance) for environmental monitoring, food safety, clinical diagnostics and laboratory applications. Further, quantitative enumeration is critical for many applications for assessing levels of contamination (for example for assessing reduction of numbers of viruses/bacteria during treatment processes for recycled water, to demonstrate required (log₁₀) reductions for regulatory compliance) and is desirable for monitoring finished drinking water and recycled water quality.

### SUMMARY

The invention disclosed herein provides a rapid and cost-effective method and/or use of a kit for culturing and detecting bacteriophage . The bacteriophage are cultured and detected within an absorptive, fibrous matrix, such as a cellulose fibre absorbent pad, which allows both growth and detection of the bacteriophage . This avoids separate steps where the bacteriophage are initially cultured or grown in liquid culture and then applied to a solid matrix or support for subsequent detection or are plated on a traditional growth medium (e.g. agar-based medium) for quantitation.

Thus, the invention broadly relates to use of an absorptive fibrous matrix, such as a cellulose fibre absorbent pad (CFAP), for culture and/or detection of viruses, bacteriophage and/or bacteria. Suitably, the absorptive fibrous matrix may facilitate quantitative enumeration of bacteriophage .

A first aspect of the invention provides a method for culture and detection of a bacteriophage in a test sample, which includes the step of culturing the bacteriophage using a bacterium that is a host for the bacteriophage within an absorptive fibrous matrix, and quantitatively enumerating the bacteriophage by detecting discrete zones of change in a detectable signal within the absorptive fibrous matrix facilitated by a detection reagent that is a substrate molecule and is selected from the group consisting of a chromogenic, colorimetric, fluorescent, electrochemical, bioluminescent, photoluminescent, and chemi luminescent detection reagent, preferably wherein the bacteriophage is detected subsequent to lysis of bacterial cells of the bacterium. Preferably, the absorptive fibrous matrix is a cellulose fibre absorbent pad (CFAP). The absorptive fibrous matrix may be used as a single layer or as a plurality of layers.

In one embodiment, the method is for detecting a bacteriophage prior to culturing and/or detecting the bacteriophage, the test sample is combined with bacterial cells that are hosts for the bacteriophage. In an embodiment, a detection reagent is added to the test sample comprising the bacterial host cells. Suitably, the test sample, bacteria and/or the detection reagent are contacted with the absorptive fibrous matrix to thereby impregnate or otherwise locate the test sample, bacteria and/or the detection reagent within and/or on the absorptive fibrous matrix.

In some embodiments, the method includes the step of inducing expression of one or more genes by the bacterial host cells, wherein the detection reagent is capable of eliciting a signal upon detecting the expression of the one or more genes, their encoded protein(s) or a metabolic or cellular product thereof. The signal may be elicited subsequent to lysis of a bacterial host cell. Preferably, the signal is colorimetric or fluorescent. Suitably, the presence of bacteriophage results in the detection reagent eliciting a signal upon detecting the expression of the one or more genes, their encoded protein(s) or metabolic or cellular products thereof, the signal may be elicited subsequent to bacterial host cell lysis.

Suitably, an embodiment includes the use of bacterial host cultures with low rates of inherent and/or non-specific cell death (such as early-mid log phase cultures or reactivated immobilised cells) and a concentration and volume of bacterial cells appropriate to the sample volume, such that bacterial cell death/lysis and detection of the signal can be specifically attributed to bacteriophage presence, infection and/or replication. Suitably, low rates of inherent and/or non-specific cell death are maintained following application of the assay suspension to the absorptive fibrous matrix, and incubation of the absorptive fibrous matrix under suitable conditions of time and temperature.

In a preferred embodiment, the method is for the quantitative enumeration of bacteriophages (e.g. coliphages) in a test sample, the method comprising:
i) combining the test sample with bacterial host cells that are early-mid log phase cultures, reactivated immobilised cells or otherwise have low rates of inherent and/or non-specific bacterial lysis to produce an assay suspension (which may include an incubation step and addition of bacterial culture media);
ii) providing a detection reagent that is, or comprises a chromogen/colorimetric or fluorescent substrate that can be hydrolysed/acted on by a bacterial enzyme or reacts with/binds to a bacterial metabolic or cellular product (e.g. protein, DNA, metabolite) that is released from the bacterial cell following bacteriophage induced lysis (e.g. the substrate chlorophenol red 6-D-galactopyranoside (CPRG) which is acted on by the *E. coli* enzyme β-galactosidase);
iii) providing an inducing agent to induce expression of the assayed bacterial enzyme or metabolic/cellular products (e.g. isopropyl-beta-D-thiogalactopyranoside (IPTG) may be added to induce expression of β-galactosidase in *E. coli*);
iv) applying the assay suspension to an absorptive fibrous matrix such as a CFAP, where the assay suspension is applied to the entire surface of the absorptive fibrous matrix, resulting in saturation or sub-saturation of the matrix (e.g. an assay volume of 1.6 mL may be applied to a CFAP with liquid holding capacity of 1.8-2 mL);
v) incubation of the absorptive fibrous matrix (with absorbed assay suspension) under suitable conditions of time and temperature (*e*.*g*. 1.5-4.5 h at 37-40 °C for detection of somatic coliphages using *E*. *coli* CN13); and
(vi) detecting the presence of bacteriophage by observing a colour change on the surface of the absorptive fibrous matrix and quantitative enumeration thereof or detection of an absence of bacteriophage by the absence of a colour change (e.g. for detection of bacteriophages using the CPRG substrate, bacteriophage presence results in colour change from yellow to red).

It will be understood that the order or sequence of steps describe above is not essential. By way of example, application of the reagents/inducing agents/substrates, bacterial cells and test sample may be varied. For example, the bacterial host cells may first be applied to the CFAP, incubated for a specified period, and then the test sample, inducing agent and/or detection reagent may be applied to the CFAP. It will be appreciated that variable volumes of assay suspension and various methods for preparing test samples (such as concentration, filtration and serial dilution) may be included.

Another aspect of the invention provides a use of a kit for culture and detection of a bacteriophage in a test sample according to a method of the invention, the kit comprising: an absorptive fibrous matrix for use as a substrate/medium for directly supporting growth and/or detection of the bacteriophage a detection reagent that is a substrate molecule and is selected from the group consisting of a chromogenic, colorimetric, fluorescent, electrochemical, bioluminescent, photoluminescent, and chemiluminescent detection reagent; and, optionally, one or more additional components selected from the group consisting of (a) a culture medium or one or more components thereof, (b) an inducing agent; (c) bacterial cells of a bacterium that is a host for the bacteriophage, wherein the bacterial cells are separate to or within or on the absorptive fibrous matrix, (d) a medium for preserving or immobilising bacterial cells, (e) RNAse; (f) positive control bacteriophage; and/or (g) one or more vessels, containers or enclosures capable of housing the absorptive fibrous matrix when in use. Preferably, the absorptive fibrous matrix is a cellulose fibre absorbent pad (CFAP). The method and/or use of a kit of the aforementioned aspects may use or comprise the absorptive fibrous matrix as one or a plurality of sheets, pads or layers, each having a surface area in the range of about 10-500 cm². In one embodiment, the absorptive fibrous matrix has a surface area in the range 17-20 cm². In another embodiment, the absorptive fibrous matrix has a surface area in the range 60-400 cm². The one or more additional reagents comprise: (a) a bacterial culture medium or one or more components thereof; and/or (b) an inducing agent. The kit may further comprise bacteria which are hosts for the bacteriophage and/or one or more reagents that may be used to immobilise or preserve the bacteria. The bacteria may be in a reversibly immobilized form, such as thermo-reversibly immobilized in gelatin, or lyophilised or dried. The kit may further comprise one or more vessels, containers or enclosures capable of housing the absorptive fibrous matrix when in use.

According to some embodiments, the test sample is, or comprises: water, inclusive of drinking water, groundwater, wastewater, wastewater effluent, recycled water, combined sewer overflows, recreational water, surface water, river water, sea water, lake water, irrigation water, swimming pool water, spa water, greywater and septic system water; other wastewater treatment by-products such as sludge and compost; food and beverage products, plant or animal tissue/material/samples; clinical isolates or samples inclusive of samples from diseased plants or animals; and/or laboratory isolates or samples.

In a preferred embodiment, the bacteriophage are coliphage. Suitably, according to this embodiment the bacteria are *E. coli, Salmonella* or other suitable enteric bacteria.

Throughout this specification, unless otherwise indicated, *"comprise", "comprises"* and *"comprising"* are used inclusively rather than exclusively, so that a stated integer or group of integers may include one or more other non-stated integers or groups of integers.

It will also be appreciated that the indefinite articles "a" and *"an"* are not to be read as singular indefinite articles or as otherwise excluding more than one or more than a single subject to which the indefinite article refers. For example, "a" cell includes one cell, one or more cells or a plurality of cells.

The term *"about"* is used herein to refer to a level of tolerance or variation in a stated amount, value or quantity. Typically, the tolerance of variation is no more than10%, 9%, 8%, 7%, 6%, 5%, 4%, 3%, 2% or 1% above or below a stated amount, value or quantity.

It will be appreciated that method steps disclosed herein are not limited to the sequential order in which the steps are recited, but may be performed in a different order to that recited while achieving the result of the method.

### BRIEF DESCRIPTION OF THE FIGURES

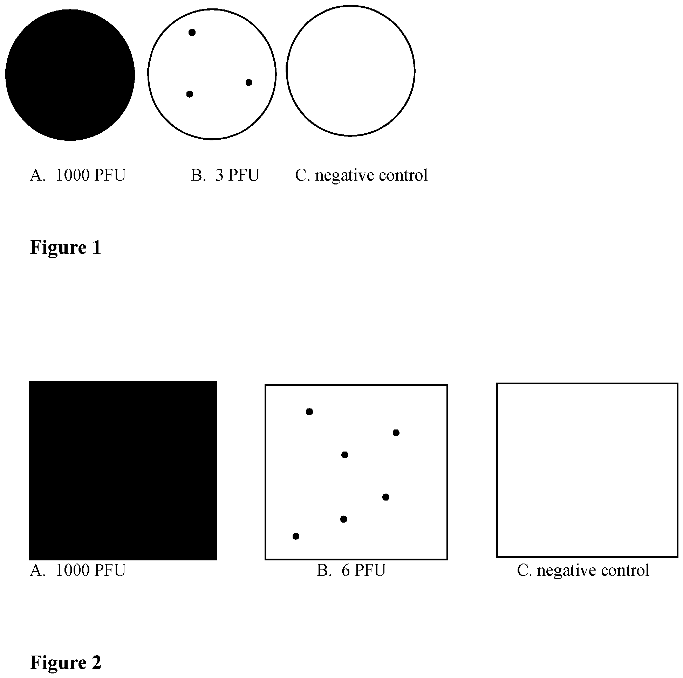

Figure 1. Example of somatic coliphage detection in 1 mL isolate dilutions on 5 cm diameter CFAP after 2 hours, where somatic coliphages are detected by turnover of CPRG substrate (colour change from yellow to red/purple is shown in this diagram as change from white to black); low numbers of coliphages are indicated by discrete zones of colour change; high numbers of coliphages are indicated by colour change across the entire surface of the CFAP; and no colour change is detected in the negative control.
Figure 2. Example of somatic coliphage detection in 10 mL isolate dilutions on 11.5x 11.5 cm CFAP after 3 hours, where somatic coliphages are detected by turnover of substrate (colour change from yellow to red/purple is shown in this diagram as change from white to black) and no colour change is detected in the negative control.

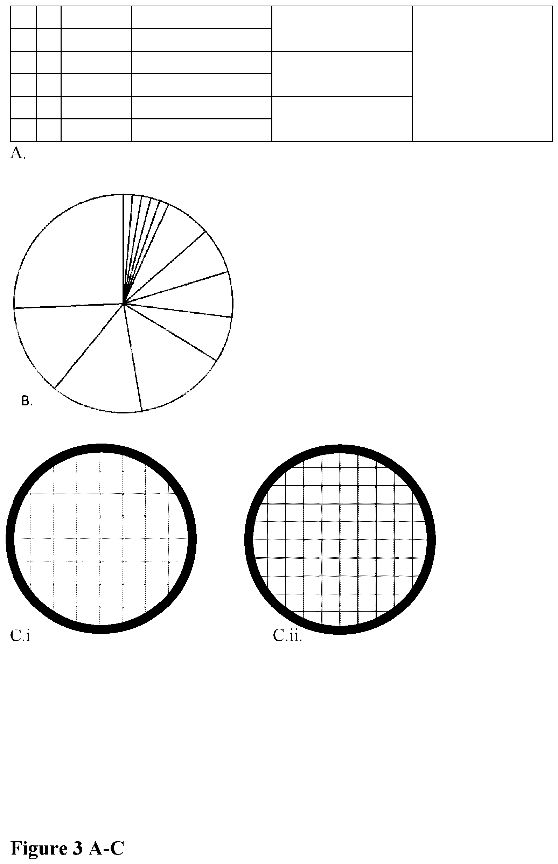

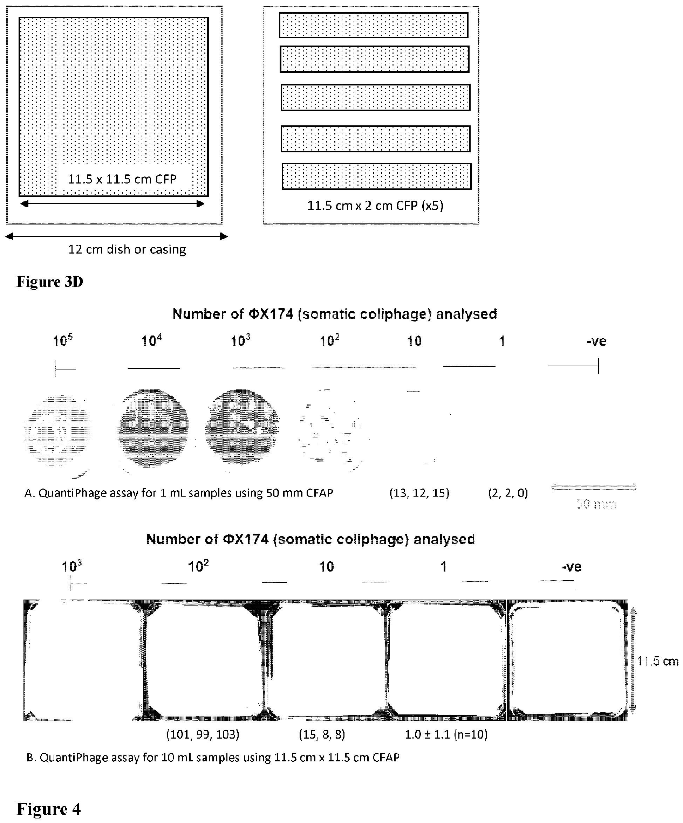

Figure 3. Examples of sectioning and patterning CFAP, which may facilitate microbial enumeration.
Figure 4. Results of experiments using the QP assay for detecting somatic coliphage isolate φX174 using (A) 50 mm diameter CFAP circles for 1 mL test samples or (B) 11.5 x 11.5 cm squares for 10 mL test samples; number of coliphages determined for triplicate replicates are indicated in parentheses.

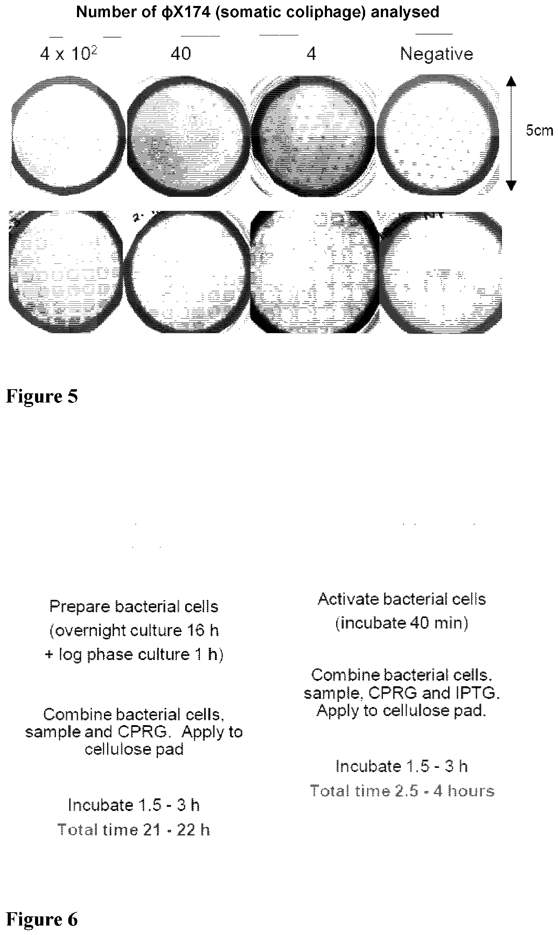

Figure 5. Use of filter paper as a support for rapid detection of coliphages; Whatman No. 1 filter paper (thickness 0.19 mm, 5 cm diameter circles), including solid ink/wax printed grids in the lower panel, only enabled an assay volume of 0.2-0.3 mL to be analysed and diffusion of positive signals was extensive; compared to 1.6 mL assay volume that may be analysed using 5 cm diameter circles of CFAP and where positive signals are localised.
Figure 6. Overview example of QP assay method for detecting coliphages.

### DETAILED DESCRIPTION

This invention is at least partly based on the discovery of the excellent properties of cellulose fibre absorbent pad materials for use as a substrate for directly supporting the rapid growth and colorimetric detection of bacteriophages/coliphages and enabling localised propagation and quantitation, and to maintain a bacterial host culture in a state of low inherent or non-specific cell lysis for the duration of the assay (i.e. resulting in the absence of background), when used with optimal assay conditions described further herein. Previously reported uses of cellulose fibre absorbent pad materials include lateral flow immunoassays (e.g. use of cellulose fibre sample pad material as a sample pad or absorbent pad to facilitate flow of the sample through the device) or as a reservoir for bacterial media to indirectly support bacterial growth on the surface of a membrane filter (e.g. cellulose fibre absorbent pad materials are used to soak up bacterial media, then a membrane filter is placed on top of the pad and the bacteria grow on the membrane surface).

A methods that involves detecting bacteria growing on the surface of a membrane filter, routinely employing sample filtration to capture bacteria onto the surface of the membrane filter, was reported by Derda et al. 2013 (Anal. Chem. 85, 7213-7220), which further included culture and solution -based assays for indirect detection of *E*. *coli* using bacteriophage and β-galactosidase-based approaches. The method described by Derda et al. 2013, involved filtering a sample through a syringe filter to capture bacteria present in a sample onto the surface of the membrane filter, then adding a bacteriophage suspension, and subsequently eluting the bacteriophages from the filter following incubation. After elution and wash steps, the eluate was assayed to infer a quantitative estimate of the number of bacteria initially present in the sample by 1) assaying bacteriophages in an agar based culture assay or 2) absorbance or fluorescence readings determined using a microplate reader. The authors reported "a nonlinear relationship between captured CFUs of bacteria and produced PFUs of phage".

In Mendez et al. 2002 (J. Virol. Methods 106, 215-224) viruses are first absorbed to a membrane filter and then eluted from the filter into a smaller volume of eluting solution-, and "...phages were concentrated by adsorption to nitrate-acetate membrane filters followed by elution according to the method first described by Sobsey et al. (1990)". Culture and detection of bacteriophages (present in the elution solution or viral concentrate) is then performed using culture and detection methods such as the double agar layer plaque assay.

A further prior art example, describes capture/immobilization of bacteriophages on biosorbents including microcrystalline cellulose beads and Disruptor filters (Minikih et al. 2003, J. Microbiol. Methods 82, 177-183). The efficiency of these phage-based biosorbents for capturing and detecting bacteria by measuring bacterial ATP released as a result of phage-induced lysis was tested, wherein: A) Genetically modified T4 bacteriophage displaying a cellulose binding molecule were used for binding to and immobilization on microcrystalline cellulose beads and; B) Wild-type T4 bacteriophage were immobilized on Disruptor filter media (nonwoven filter media based on alumina nanofibers). The method disclosed by Minikih et al. (2003) involved mixing a bacterial sample (0.3 mL) with the phage-based biosorbent in a bacterial culture medium (0.1 mL), and after sample incubation an aliquot of the solution was assayed for ATP released due to bacterial lysis, wherein measurement of the detection signal was in solution using a luminometer.

The present invention relates to bacteriophage enumeration in any application, including environmental monitoring, food/beverage safety and clinical applications, although without limitation thereto. These applications may include assessing a test sample (e.g. water/wastewater, food/beverage product or clinical sample) or detecting laboratory/clinical isolates. Accordingly, in particular embodiments, the present invention relates to a rapid method for detecting/enumerating bacteriophage in a sample as an indicator of faecal viruses/bacteria, faecal pollution and/or microbial quality in environmental monitoring (e.g. water/wastewater) and food safety applications. The present invention also relates to enumeration of bacteriophages that are used as a therapeutic agent for treatment of bacterial infections in clinical applications or to kill target bacteria in industrial applications (e.g. in agriculture and fisheries). For enumeration of bacteriophages, bacterial host cells may be freshly cultured or immobilised or preserved bacterial cells wherein methods including (but not limited to) reversible-gelation, lyophilisation or drying may be used to preserve bacteria. Rapid tests for bacteriophages can enable water and food safety to be monitored more actively to better protect public health and can improve outcomes in clinical applications. Simple, rapid and inexpensive tests for bacteriophages can also promote more routine monitoring of microbial quality in wider applications and are highly demanded by relevant industries and laboratories that perform large scale testing. The disclosed methods have advantages compared to the prior art, in terms of rapidity, ease of use, low cost and non-inclusion of genetically modified organisms.

Thus, the invention broadly relates to use of an absorptive fibrous matrix, such as a cellulose fibre absorbent pad (CFAP), for culture and quantitative detection of bacteriophage.

As generally used herein, an *"absorptive fibrous matrix"* may be any absorptive fibrous substrate, material, membrane, support or other matrix that allows or facilitates growth and/or detection bacteriophage . Suitably, the absorptive fibrous matrix is not, or does not comprise, paper such as filter paper. Suitably, the absorptive fibrous matrix allows or facilitates a liquid test sample comprising bacteriophage to diffuse, permeate, distribute or otherwise be absorbed into or by the fibrous matrix.

In an embodiment, application of a liquid test sample of a suitable volume to the absorptive fibrous matrix results in saturation or sub-saturation of the absorptive fibrous matrix, wherein sub-saturation refers to application of a liquid volume just below the liquid holding capacity of the absorptive fibrous matrix, and the absorptive fibrous matrix functions to limit diffusion of the liquid test sample.

Typically, the bacteriophage are directly retained by, within and/or on the absorptive fibrous matrix during culture and are detected directly on or within the absorptive fibrous matrix. Generally, the matrix may be in the form of a layer, sheet, pad, mat, wad or other structure that has a generally planar profile. Suitably, the matrix may have a thickness in the range of about 0.2 mm to about 5 mm, preferably about 0.5 mm to about 4 mm or more preferably about 0.7 mm to about 3 mm. In general embodiments, the absorptive fibrous matrix may include cellulosic materials such as cellulose fibres (e.g. cotton linter), glass fibre, modified/polyester, nylon, nitrocellulose, mixed cellulose esters, cellulose acetate, cellulose nitrate, PTFE, natural, synthetic or chemical polymer fibres, although without limitation thereto. In embodiments where the absorptive fibrous matrix comprises cellulose, a particular embodiment may be a cellulose fibre absorbent pad (CFAP). Non-limiting embodiments of different types of CFAP are commercially available from Millipore^{®} (cellulose fibre sample pad sheet material #CO83, thickness (T) = 0.83 mm, grammage (G) = 291 g/m²; #C248, T = 2.48 mm, G = 702 g/m²) or Advantec^{®} (#PD-47B, absorptive cellulose fibre pads, 47-50 mm in diameter, supplied pre-sterilised in Petri dishes) or GE^{®} (#VF2, T = 0.785 mm, water absorption (WA) = 86.2 mg/cm²; CF5, T = 0.954 mm, WA = 99.2 mg/cm²; CF6, T =1.45 mm, WA = 136.3 mg/cm²; CF7, T = 1.87 mm, 252.3 mg/cm²).

Although cellulosic materials may generally be useful according to the invention, CFAP is a preferred material that has greater thickness, grammage, rigidity and bed volume compared to other cellulosic materials such as paper (wherein grammage refers to mass per unit area (g/m²) and bed volume refers to the volume of air held in the pores of the material per unit area (e.g. mL/cm²), such that a greater sample volume is required to saturate a material with a higher bed volume). Such differences between CFAP and paper may be associated with differences in functional properties of the materials in the method disclosed herein, notably diffusion of assay signals and sample volumes that may be analysed.

Compared to other cellulosic materials such as paper/filter paper, CFAP has greater rigidity/stiffness, thickness, density/grammage and bed volume/liquid holding capacity. CFAP typically has thickness of about 0.4-2.5 mm, grammage of about 180 g/m² to about 800 g/m², and liquid holding capacity of about 0.06 mL/cm² to about 0.5 mL/cm², although without limitation thereto. Paper/filter paper typically has thickness of about 0.16-0.4 mm, grammage of about 68 g/m² to about 185 g/m² and liquid holding capacity of about 0.01 to 0.03 mL/cm². An example of differences in bacteriophage detection using CFAP and filter paper is shown in Figure 4 and Figure 5. The differing properties of CFAP compared to paper/filter paper, critically support quantitative bacteriophage enumeration equivalent to the standard plaque assay methods in the disclosed CFAP-based method for large sample volumes, such as 1 mL and 100 mL volumes.

Thus, in some embodiments the absorptive fibrous matrix is not paper, such as filter paper.

Suitably, the absorptive fibrous matrix is sterilized or sterilizable.

The absorptive fibrous matrix may be used as a single layer, sheet, pad or mat or may be used as a plurality of layers, sheets, pads or mats, such as a "stack" of layers, sheets, pads or mats.

The absorptive fibrous matrix may be used in any of a variety of different shapes and sizes. Generally, the absorptive fibrous matrix may have a surface area in the range of about 10-500 cm², 15-450 cm², 20-400 cm², 25-350 cm², 30-300 cm², 40-250 cm², 50-200 cm², 70-180 cm², 80-150 cm², 100-120 cm², or any size or size range between these stated values.

Typically, smaller test sample volumes will require a lower surface area of absorptive fibrous matrix and larger test sample volumes will require a larger surface area of absorptive fibrous matrix.

In one general embodiment, for 1 mL test samples the absorptive fibrous matrix has a surface area in the range of about 10-50 cm², or preferably about 17-20 cm².

In another general embodiment, for 10 mL test samples the absorptive fibrous matrix has a surface area in the range of about 100-500 cm² or preferably about 100-250 cm².

In one non-limiting example, approximately 47-50 mm diameter (*i.e* about 17-20 cm²) absorptive fibrous matrix circles may be used, such as for analysis of 1 mL viral concentrates/test sample. In another non-limiting, example, 11.5 cm x 11.5 cm (*i.e* about 132.25 cm²) absorptive fibrous matrix squares or 10 cm x 15 cm (*i.e* about 150 cm²)absorptive fibrous matrix rectangles may be used, such as to analyse 10 mL test samples (such that multiple squares can be used for 100 mL samples). In yet a further non-limiting example, approximately 87 - 90 mm (*i.e* about 59-65 cm²) or 140 mm diameter (*i.e* about 154 cm²) circles may be used for analysis of viral, bacteriophage or bacterial concentrates/test sample. Other embodiments include, 10 cm x 10 cm (100 cm²) squares and 11.5 cm x 2 cm (23 cm²) strips.

In yet a further example, varying numbers or sizes of CFAP may be combined for quantitation (such as Figure 3 where the absorptive fibrous matrix is cut into segments or segments are created by patterning). In this embodiment the absorptive fibrous matrix may be patterned with a grid pattern or compartments/sections, where the pattern is printed using a method such as solid ink/wax printing, lithography or laser treatment; or whereby compartments/sections are formed by any other means (such as scoring or cutting).

Suitably, the absorptive fibrous matrix may be encased, housed or enclosed by any means, such as a vessel, container or other enclosure. In particular embodiments, the vessel, container or enclosure may be or include a Petri dish or other sterile or sterilisable dish, container, single or multi-well tray or plate, plastic pocket or bag or overlay such as a transparent film/plastic. Such vessels, containers or other enclosures may have removal or dissolvable sections to facilitate access to the absorptive fibrous matrix, reagents, media or other materials housed, encased or otherwise located therein.

As hereinbefore described, bacteriophage bacteria are directly retained by or within the absorptive fibrous matrix during culture and are detected directly on or within the absorptive fibrous matrix.

As broadly used herein the term *"bacteriophage"* includes and encompasses any virus that is capable of infecting and replicating in a bacterium. Bacteriophage may have a DNA or RNA genome comprising single-stranded or double-stranded DNA or RNA. The genome is typically packaged or encapsulated by proteins encoded by the bacteriophage genome. Bacteriophage are frequently non-enveloped or may be enveloped (*e*.*g* encapsulated by bacterial host-derived lipids, glycolipids and/or lipoproteins). Bacteriophage may exhibit a lytic cycle or a lysogenic cycle (*e*.*g* "temperate" phage) associated with replication in a bacterial host. Particular, non-limiting examples of bacteriophage include *Caudovirales* such as *Myoviridae, Siphoviridae* and *Podoviridae,* and other families including *Tectiviridae, Plasmaviridae, Corticoviridae, Microviridae, Inoviridae, Leviviridae,* and *Cystoviridae,* although without limitation thereto. In an alternative view, the term *"bacteriophage"* is extended to include and encompass any virus that is capable of infecting and replicating in a prokaryotic cell. Thus, viruses which infect Archaea have also been referred to as bacteriophages, including *Ligamenvirales* such as *Lipothrixviridae* and *Rudiviridae* and other families such as *Ampullaviridae, Bicaudaviridae, Clavaviridae, Fuselloviridae* and *Globuloviridae,* although without limitation thereto.

By way of example only: *Myoviridae* are bacteriophages with a double-stranded (ds) DNA genome, have an icosahedral capsid with complex contractile tails and include, as examples, bacteriophage Mu, P1, P2, and T4 and the "T4-like" bacteriophage; *Siphoviridae* have a dsDNA genome, are icosahedral and characterized by long, non-contractile tails and include, as examples, λ, hk022, T5, and BF23 bacteriophage; *Podoviridae,* have a dsDNA genome, are icosahedral and characterized by short, non-contractile tails and include, as examples, bacteriophages N4, P22, T3, and T7; *Tectiviridae* have a dsDNA genome, are icosahedral with an internal membrane, and includes, as an example, bacteriophage PRD1; *Plasmaviridae* have a dsDNA genome, are quasi spherical with an internal membrane, and includes, as an example bacteriophage L2; *Corticoviridae* have a dsDNA genome, are icosahedral with an internal lipid-containing membrane, and includes bacteriophage PM2; *Microviridae* have a single-stranded (ss) DNA genome, are icosahedral and include, as examples, bacteriophage such as G4 and φX174; *Inoviridae* have a ssDNA genome and comprise rod-shaped or filamentous bacteriophage and include, as an example bacteriophage M13; *Leviviridae* have a ssRNA genome, are icosahedral and includes bacteriophage MS2; and *Cystoviridae* are dsRNA bacteriophages with an envelope, and includes bacteriophage φ6.

It will be appreciated that according to the invention, the bacteriophage is typically isolated from any artificial or natural environment or source. For bacteriophage, this may be wherever a susceptible bacterial host may be present, although bacteriophage may also be isolated in the absence of the corresponding host. Non-limiting examples of environmental or natural sources include contaminated or infected water systems, aquaculture systems, industrial waste, sewage, mining waste, soil, medical waste, water and human and other animal materials such as sputum, wound fluid, urine, blood, faeces and throat swabs, although without limitation thereto.

Suitably, the bacteriophage are capable of infecting, and propagating in and replicating in a bacterium and/or lysing a bacterium. It will be appreciated that bacteriophage referred to herein may be capable of infecting bacteria inclusive of gram-positive and gram-negative bacteria, rods, cocci, non-motile and motile bacteria, anaerobes, facultative anaerobes, aerobes and photosynthetic bacteria, although without limitation thereto. It will also be appreciated that bacteria may be "naturally occurring" bacteria or genetically-modified (GM) bacteria. By way of example, the bacteria may be of a genus or other taxonomic group such as *Staphylococcus, Bacillus, Yersinia, Hemophilus, Helicobacter, Streptococcus, Neisseria, Klebsiella, Brucella, Chlamydia, Rickettsia, Bordatella, Clostridium, Listeria, Legionella, Vibrio, Enterobacter, Pasteurella, Bacteroides, Campylobacter, Lactococcus, Diplococcus, Pseudomonas, Borrelia, Citrobacter, Corynebacterium, Moraxella, Neisseria, Escherichia, Salmonella, Propionibacterium, Shigella or Enterococcus,* although without limitation thereto.

Bacteriophages referred to as *"coliphages"* infect *E. coli* and other *Enterobacteriaceae* bacteria such as *Klebsiella, Salmonella* and *Shigella,* although without limitation thereto. Coliphages include somatic coliphages (*Myoviridae,Siphoviridae, Podoviridae* and *Microviridae*) and F+ coliphages (*Inoviridae andLeviviridae*)*.*

In an embodiment, bacterial cultures used for bacteriophage replication and detection have inherently low rates of cell death and/or non-specific cell lysis. Typically, the method uses a concentration and volume of bacterial cells that is appropriate for the analysed sample volume. In one embodiment, freshly prepared bacterial cultures are used for culture and detection of bacteriophages. In some embodiments, bacteria may be prepared by inoculating bacterial culture media with bacterial cells from a 16-18 hour culture, and then incubating the culture with shaking at an appropriate temperature for an appropriate time, to produce an early-mid log phase culture (e.g. for *E*. *coli* CN13 and *E.coli* 70089, the culture may be incubated for 40 - 90 minutes, 37-40 °C, 180-200 rpm).

In other embodiments, bacterial host cells may be provided by activating bacterial cells that are in an immobilized or preserved form, wherein the cells have inherently low rates of cell death and/or non-specific cell lysis. These may include bacteria prepared by:
(i) lyophilisation or drying, such as in a lyophilisation vial/container or on the absorptive fibrous matrix, using standard procedures and cryoprotectants;
(ii) immobilisation wherein reversible-gelation (e.g. temperature-, pH- or chemical-dependent reversible gelation) is used to immobilised bacteria in a gel or gel-like material and then at the time of use the gel is reverted to a liquid for usage of the bacterial host cells;
(iii) immobilisation wherein bacteria are applied/incorporated in a gelatinous film or layer, on or within the absorptive fibrous matrix (such as application of bacteria to the surface of the CFAP in a reversible or non-reversible gel layer);
(iv) immobilisation wherein bacteria are immobilised in a layer of gelatinous or other material, and the absorptive fibrous matrix is placed atop of the layer;
(v) thermo-reversible immobilisation of bacterial cells using a gelatin solution.

For example, thermo-reversible immobilisation of bacterial cells may include:
Preparation of a gelatin solution (e.g. 3% gelatin, 0.15% peptone and 0.1% beef extract, where the gelatin is dissolved with heat and the solution is autoclaved at 121 °C for 20 min), which may include the use of other additives;
i) Preparation of a log phase bacterial host cell culture (e.g. 30 mL, OD520 = 0.2-0.8), centrifugation of the bacterial host cells (e.g. 3500 rpm, 5 min, 20 °C for *E*. *coli* CN13, or3000 rpm, 5 min, 4 °C for *E. coli* 700891 cells) and resuspension of bacterial cells in gelatin containing solution (e.g. 15 mL LB⁺[+ antibiotic] and 15 mL 3% gelatin solution);
ii) Aliquoting the bacteria-gelatin solution into vessels (e.g. 1.5 mL screw cap tube or 10 mL tubes or 7 mL McCartney Bottle) and storing at 4-8 °C (whereby a gel is formed that immobilises the bacteria), and may include subsequent overlay of the gel with a material that may limit oxygen diffusion such as LB+ media or paraffin oil;
iii) For use in rapid coliphage assays (e.g see the QP assay described hereinafter), the gel/immobilised cells is heated (e.g. at room temperature (~25 °C) or 37 - 40 °C) and reverts to a liquid; fresh bacterial media is added to an aliquot of the bacterial cells, which are then reactivated/commence growth (e.g. 450 µL LB⁺ (+ antibiotic) may be added to 150 µL cells, the suspension is incubated at 37-40 °C for 40 min, in a horizontal position and without shaking and may be mixed by inversion every 15 min; cells and media for multiple assays may combined for the incubation).

Alternatively, following gel formation in step (iii), the bacterial media may be applied atop or over the gel, such that it does not need to be subsequently added at the time of warming/use of the gel-bacteria in step iv).

The bacterial cells may then be used in the method disclosed herein.

It will also be appreciated that the gelatin immobilisation method may be altered by using different concentrations of gelatin, including other gel forming agents (e.g. alginate, silicate, hydrogels, chitosan, methyl cellulose, gums, α-carraggenan), or additives that improve cold tolerance or survival (e.g. trehalose, 0.25-2.5% sodium glutamate) or specific culture conditions (e.g. high salt or minimal media) may be used to promote survival of immobilised bacteria.

It will be appreciated that culture of bacteria may be performed using any suitable bacterial growth media. The media may be supplemented with antibiotics appropriate for the bacterial host cell type. A non-limiting example is LB+ media. By way of example a growth medium suitable for *E. coli* CN13 is LB+: 10 g tryptone and 5 g yeast extract per litre, 0.27 M NaCl, 25 mM MgSO4, 5 mM CaCl2, supplemented with nalidixic acid antibiotic.

It will be appreciated that the invention relates to quantitative enumeration of bacteriophage.

In some embodiments, quantitative enumeration may be facilitated by application of a liquid test sample of a suitable volume that results in saturation or sub-saturation of the absorptive fibrous matrix, such as CFAP, wherein sub-saturation refers to application of a liquid volume just below the liquid holding capacity of the absorptive fibrous matrix, and the absorptive fibrous matrix functions to limit diffusion of the liquid test sample.

Detection, measurement and/or enumeration of the bacteriophage is facilitated by a detection reagent. Such detection reagents may be chromogenic, colorimetric, fluorescent, electrochemical, bioluminescent, photoluminescent or chemiluminescent detection reagents.

Non-limiting examples of detection reagents include chlorophenol red 6-D-galactopyranoside and 4-methylumbelliferyl β-D-galactopyranoside.

In a preferred form, the detection reagent is not directly coupled, affixed, conjugated or otherwise bound or immobilized (e.g. chemically coupled, affixed, conjugated or otherwise bound or immobilized) to the adsorptive fibrous matrix.

In a further embodiment, specific detection of F+ DNA coliphages may be achieved by inclusion of RNase in the assay (enabling determination of F+ RNA coliphage numbers) and/or means for the specific detection of different types of bacteriophages may be included (e.g. discrimination of different genotypes of F+ coliphages by using anti-sense RNA linked technology).

In another further embodiment, multiple types of bacteriophages in a test sample may be detected by combining a number of different bacteriophage specific host bacterial cells and different host specific chromogens/substrates or by using a single chromogen that produces a different colour dependent on the specific bacterial host cell enzyme, metabolic or cellular products and/or bacteriophage present.

A non-limiting example of this embodiment may include:
(I) *E. coli* with CPRG substrate to detect coliphages and *Enterococcus* spp. With *p*-nitrophenyl-β-D-glucopyranoside substrate to detect *Enterococcus* phage; or
(II) Use of propylene glycol in an assay for detection of phages of *E. coli* and *Salmonella* spp., where lysed *E*. *coli* appear blue/green and lysed *Salmonella* spp. appear red.

In some embodiments, the method and use of a kit disclosed herein may use, or provide, an inducing agent.

As broadly used herein, an *"inducing agent"* is any molecule capable, of triggering, promoting, eliciting or otherwise inducing the expression of one or more bacterial host cell genes. Suitably, the detection reagent facilitates detection of expression of the one or more induced genes, one or more proteins encoded by the genes, or one or more metabolites as hereinbefore described.

By way of example, the one or more proteins may be an enzyme or other catalytically active protein that can convert a detection reagent substrate molecule to thereby elicit a detectable signal. A non-limiting example of an inducing agent isisopropyl-beta-D-thiogalactopyranoside (IPTG), which binds to the lac repressor, allowing for expression of genes in the lac operon, such as β-galactosidase. The induced β-galactosidase can then catalytically act upon or convert a detection reagent substrate. For example, the detection reagent substrate chlorophenol red 6-D-galactopyranoside (CPRG) and inducing agent isopropyl-beta-D-thiogalactopyranoside (IPTG) may be used to produce a detectable red signal. Typically, a detectable signal is elicited where induced bacterial host cells have been lysed by the bacteriophage. Accordingly, the presence of a detectable signal is an indicator of the presence of bacteriophage. Accordingly, the absence of a detectable signal is an indicator of the absence of bacteriophage. It will also be appreciated that the detectable signal may facilitate quantitation, or semi-quantitation of bacteriophage in the test sample.

It will also be appreciated that images of the colour change may be captured (e.g. using a smart phone camera), and may be analysed by an application (app) or image analysis software for earlier detection or automated calculation of the number of bacteriophages present in the sample and/or automated relay of results. It will also be appreciated that QP assay method may be performed partially or completely using an automated analysis system, method or device, that may also include continuous automated monitoring and use of extended lengths of absorptive fibrous matrix.

The absorptive fibrous matrix CFAP disclosed herein is superior to other materials such as paper/filter paper as a substrate as the higher thickness, density/grammage and bed volume of the absorptive fibrous matrix enables analysis of greater sample volumes. Furthermore, absorptive fibrous matrix disclosed herein results in limited reagent diffusion when appropriate sample volumes are applied to most of, or substantially the entire surface of the absorptive fibrous matrix and optimal assay conditions disclosed herein are used (such as optimal bacterial host cell preparation methods, bacterial cell growth stage, bacterial cell concentration, reagent concentrations and assay time). This enables quantitative detection (e.g. for low numbers of bacteriophages in a dilution series and/or samples containing less than approximately 1000 PFU/100 mL) in sample volumes relevant for water/food quality analysis, that are routinely analysed in the plaque assay in standard methods of analysis and in regulatory guidelines (e.g. 1 mL and 100 mL). More specifically, the disclosed methods enable individual bacteriophages to be detected and quantitated by supporting localised bacteriophage propagation on or within the absorptive fibrous matrix, such as CFAP, that results in discrete zones of colour change (equivalent to plaques), due to bacterial host cell lysis and turnover of a colorimetric substrate in CFAP-based assays (in as little as 2 hours for 1 PFU somatic coliphages, and up to about 4.5-5 hours). The production of characteristic zones of colour change (equivalent to plaques) using the disclosed assay, which increase rapidly in size and intensity within hours, also means that overnight confirmation of results is not required (as in other liquid assays). Furthermore, an absence of colour change (and background) is observed in the absence of bacteriophage using the disclosed assay method.

The production of characteristic zones of colour change (equivalent to plaques) in CFAP-based assays is a distinct advantage for quantitation compared to liquid-based assays, as individual bacteriophages/plaques (or bacteria) are not discriminated in liquid-based assays. The production of localised regions of colour change and shorter assay time (generally 1.5-5 hours) is also a potential reason that background is not observed using the disclosed method (as it may be in other liquid assays where colour change must be effected through large volume samples and/or more lengthy incubation times are required) and the need for GMO/selected strains or enzyme substrate immobilisation to reduce background is negated. The disclosed method also does not require multistep laboratory procedures involving equipment such as centrifuges and filtration that are frequently used in liquid assays (Izjerman et al. 1993; Stanek et al. 2001). Further, use of the CFAP-based method disclosed herein supports more rapid detection of low numbers of bacteriophages (1 PFU, in volumes such as ≥ 1 mL and ≥10 mL and 100 mL) compared to liquid-based assays, and overnight confirmation of results is not required. The disclosed method is the only known rapid method to provide directly equivalent results to the conventional/standard plaque assay method, wherein for quantitative applications the CFAP functions similarly to soft agar in the plaque assay, to limit diffusion of assay constituents and support localised bacteriophage propagation, when appropriate assay volumes and numbers of bacteriophages are applied to the entire surface of the CFAP. Test samples with low numbers of bacteriophages produce localised zones of colour change (equivalent to plaques in the plaque assay) and high numbers of coliphages produce colour change across the entire surface of the CFAP (equivalent to clearing of the lawn of bacteria in the plaque assay). In addition to providing rapid results, the CFAP-based assays have additional advantages compared to conventional assays using agar, as much less time (if any) is required for assay preparation/performing the assay, and laborious procedures of autoclaving and tempering of agar and lengthy overnight incubations are not needed. This is of particular value for laboratories that perform many tests per day.

Accordingly, one embodiment of the present invention relates to a method that can be used for the rapid, quantitative enumeration of bacteriophages (for example, but not limited, to coliphages) referred to herein as a "QuantiPhage (QP)" assay. The QP assay method, which is described further in the examples below, relates to the application of specific assay volumes (including optimal sample volumes and concentrations/growth stage of host bacterial cells and concentration of reagents), to the entire surface of a sterile absorptive fibrous matrix, such as CFAP, and incubating for specified times and temperatures. Furthermore, bacteriophage quantitation is supported by the application of optimal assay volumes that result in saturation or sub-saturation of the absorptive fibrous matrix such as CFAP (wherein sub-saturation refers to application of an assay volume just below the volume holding capacity of the matrix or just below a volume that results in saturation), wherein the absorptive matrix functions similarly to soft agar that is used in conventional plaque assays to restrict diffusion of assay constituents. The method involves combining a test sample (or laboratory/clinical isolate) with appropriate bacterial host cells and a chromogenic substrate for a bacterial host cell enzyme, and application of the mixture to CFAP material or similar absorptive material where 1) expression of the enzyme may be induced with an inducing agent and additives that promote bacterial host cell lysis may be included; 2) bacterial host cells of optimal concentration (OD520) and growth state are used; 3) optimal concentrations of inducing agents, substrates and bacterial culture media are used; 4) optimised/specified assay and sample volumes are applied to an absorptive fibrous matrix of specified size to limit signal diffusion; and 5) the CFAP material is incubated for a pre-determined time and temperature, and then bacteriophages are detected visually as a colour change due to bacteriophage induced bacterial host cell lysis, release of the bacterial enzyme and turn-over of the chromogenic enzyme substrate; and there is no observable colour change in the absence of bacteriophage.

For example, for detection of somatic coliphages, a water sample may be combined with prepared *E. coli* CN13 cells, the substrate chlorophenol red 6-D-galactopyranoside (CPRG), inducing agent isopropyl-beta-D-thiogalactopyranoside (IPTG) and bacterial culture media and the suspension applied to CFAP and incubated at 37-40 °C for 1.5-2 hours or 2.5-4.5 hours. In other embodiments, any sequence of combining and applying reagents/cell-suspension/test-sample to CFAP may be used. The said QP method may comprise analysing test samples for the detection of bacteriophages (including, but not limited to, coliphages) as an indicator of faecal contamination/faecal virus contamination or microbial quality. Such test samples include, but are not limited to, water (drinking water, groundwater, wastewater, wastewater effluent/recycled water, combined sewer overflows, recreational water [e.g. surface water, river water, sea water] and irrigation water), other wastewater treatment by-products (such as sludge and compost) and food/beverage products (e.g. fresh fruit and vegetables, packaged foods/beverages, meat and seafood such as shellfish). The QP assays may be used for water samples without or with concentration procedures (that are used to recover viruses from large volume samples into smaller volumes) and filters used for concentrating samples may be incorporated into the assay. The said QP method may also comprise detecting bacteriophages used as a therapeutic agent (e.g. testing of clinical isolates or samples) or for laboratory techniques (such as phage display).

The QP method for detecting bacteriophages may be performed using freshly cultured or immobilised/preserved bacterial host cells, wherein the bacterial cultures have low inherent rates of cell death and/or non-specific cell lysis, and wherein an appropriate concentration and volume of bacterial cells for a given sample volume is used. Freshly cultured bacterial cells may be prepared by firstly establishing a liquid bacterial culture by incubating the culture overnight (~18 hour) with shaking and then inoculating additional media with the overnight culture and incubating for a further period to producean early-mid log phase culture (e.g. ~ 1 hour for *E*. *coli* CN13, 37-40 °C, 180 rpm, OD520= 0.3-0.4 for analysis of 1 mL samples; and ~1.5 hour forE. *coli* CN13, 37-40 °C, 180 rpm, OD520 ~ 1.0 for analysis of 10-100 mL samples; OD520 = 0.3-1.0 is equivalent to about 10⁷-10¹⁰ cells/mL). An example of a bacterial culture medium is LB+ medium (10 g tryptone, 5 g yeast extract, 0.27 M NaCl and 25 mM MgSO4 per litre; amended with sterile 5 mM CaCl₂ and antibiotics appropriate for the bacterial host cell after autoclaving). Immobilised/preserved bacteria may be prepared using common lyophilisation procedures (e.g. in a lyophilisation vial or on the surface of the CFAP type material) or reversible-gelation procedures (including, but not limited to, thermo- and pH-reversible gelation).

In one embodiment of the QP assay, 1 mL test samples (which may include serial dilutions of viral concentrates) are combined with bacterial host cells (e.g. 600 µL), chromogenic substrate (and an enzyme inducing agent or other additives as appropriate to the host cell) and bacterial culture media and applied to ~4.7-5 cm diameter CFAP circles (such as Advantec^{™} CFAP supplied pre-sterilised in petri dishes or cut from Millipore CFAP #C083, thickness 0.83 mm, autoclaved, dried and placed in 5 cm diameter Petri dishes) and incubated (e.g. 37-40°C, 1.5-4.5 hours for somatic coliphages and 2.5-4.5 hours for F+ coliphages; Figure 1); this embodiment enables equivalent sample volumes to be analysed and provides equivalent results to the double agar layer plaque assay (e.g. US-EPA Method 1602). In another embodiment, 10 mL test samples are combined with bacteria and reagents as described previously and applied to 11.5 cm x 11.5 cm squares of CFAP (e.g. cut from Millipore CFAP #CO83, thickness 0.83 mm, autoclaved, dried and placed in 12 x 12 cm Petri dishes; Figure 2); 100 mL samples may thus be analysed without concentration by using 10 x (11.5 cm x 11.5 cm) squares; this embodiment enables equivalent sample volumes to be analysed and provides equivalent results to the single agar layer plaque assay (e.g. US-EPA Method 1602). Alternative CFAP sizes for analysing 100 mL samples include, for example, multiple 10 cm x 10 cm squares, 10 cm x 15 cm rectangles, or 9 cm or 14 cm diameter circles. In yet a further embodiment, large volume samples (e.g. 10 - 100 mL) are applied to a compartmentalised/sectioned CFAP sheet, wherein the compartments/sections are of differing size and can thus be used for quantitation similarly to compartmentalised wells systems (Figure 3). In yet a further embodiment, CFAP is printed with a grid pattern to limit reagent diffusion, where the grid pattern is printed using a method such as solid ink/wax printing, lithography or laser treatment (e.g. Figure 3). In yet a further embodiment, target detection may be facilitated by the use of fluorescent substrates and/or alternative detection technologies.A further embodiment of the QP assay relates to multiplex-type applications. In this embodiment, different types of bacteriophages may be detected in one assay, by using different bacterial host cells and specific enzyme-chromogen/s. For example, a combination could include (but is not limited to): 1) *E. coli* + CPRG substrate to detect coliphages and *Enterococcus* spp. + *p*-nitrophenyl-β-D-glucopyranoside substrate to detect *Enterococcus* phage; or 2) use of propylene glycol in an assay for detection of phages of *E. coli* and *Salmonella* spp., where lysed *E. coli* appear blue/green and lysed *Salmonella* spp. appear red. In further embodiments, the method may further comprise inclusion of means for identifying different types of bacteriophages present, e.g. genotypes of F+RNA coliphages (for example using anti-sense RNA linked beacons) or F+ DNA and F+ RNA coliphages by inclusion of RNase in the assay.

According to the invention disclosed herein, test samples may include, water (for example drinking water, groundwater, wastewater, wastewater effluent, recycled water, combined sewer overflows, recreational water, surface water, river water, sea water, lake water, irrigation water, swimming pool water, spa water, greywater, septic systems); Water samples may be un-concentrated samples or concentrated samples (using any concentration method), or bacteriophages/microorganisms may be recovered from a water sample for example onto a filter that is included in the method of the invention. Additives may be included to neutralise effects of disinfectants or other contaminants/chemicals/materials (such as sodium thiosulphate for neutralisation of chlorine). Samples may be pre-treated using any method for removal of undesired organisms or debris (e.g. pre-filtration).

In other embodiments, test samples may include:
Other wastewater treatment by-products (such as sludge and compost);
Food and beverage products (for example fresh fruit and vegetables, meat and seafood such as shellfish, beer, wine, juice and milk);
Plant or animal tissue/material/samples;
Clinical isolates or samples inclusive of samples from diseased plants or animals Laboratory isolates or samples;
Thus, the method and kit disclosed herein may be suitable for assessing:
   faecal contamination or faecal virus contamination of a test sample;
   microbial and/or virological quality of the test sample;
   virus removal/disinfection during water/wastewater/drinking water treatment processes;
   detection of the survival or presence of bacteriophage strains used as a therapeutic agent, industrial tool or laboratory tool.

So that the invention may be readily understood and put into practical effect, reference is made to the following non-limiting examples.

### EXAMPLES

### Example 1: Preparation of gelatin-immobilised E. coli CN13 bacterial host cells

### 1. Preparation of gelatin-immobilised E. coli CN13 bacterial host cells for 1 mL assay:

a. Preparation of a gelatin solution (3% gelatin, 0.15% peptone and 0.1% beef extract) by dissolving the gelatin with heat and autoclaving the solution at 121 °C for 20 min. The gelatin solution may also be amended with other additives (e.g. 0.5-2.5 % sodium glutamate, sodium silicate, trehalose);
b. Preparation of an overnight starter bacterial culture by inoculating 5 mL LB+ (amended with nalidixic acid, LB+N) with *E*. *coli* CN13 from a glycerol stock or plate and incubation at 37 °C, 180 rpm for 16-18 h (overnight starter cultures described below are prepared similarly);
c. Preparation of a log phase *E. coli* CN13 culture by inoculating media (30 mL LB⁺N) with 300 µL - 600 µL overnight starter culture and incubation at 37- 40 °C, 180 rpm until OD520 = 0.23 (~60 min); d. Centrifugation of the log phase culture (3500 rpm, 5 min, 20 °C);
e. Resuspension of the cell pellet in 15 mL LB⁺N and 15 mL 3% gelatin solution; and
f. Aliquoting of the gelatin-bacteria suspension into a 1.5 mL screw cap tube with o-rings or 10 mL tube or 7 mL McCartney Bottle, and storage at 4-8 °C for up to 2-6 months.

### Example 2: Preparation of gelatin-immobilised E. coli CN13 bacterial host cells

### 2. Preparation of gelatin-immobilised E. coli CN13 bacterial host cells for 10 mL assay:

a. Preparation of a gelatin solution (3% gelatin, 0.15% peptone and 0.1% beef extract) by dissolving the gelatin with heat and autoclaving the solution at 121 °C for 20 min; The gelatin solution may also be amended with other additives (e.g. 0.5-2.5 % sodium glutamate, sodium silicate, trehalose);
b. reparation of a starter bacterial culture by inoculating 5 mL LB+ (amended with nalidixic acid, LB+N) with *E. coli* CN13 from a glycerol stock or agar plate and incubation at 37 °C, 180 rpm for 16-18 h;
c. Preparation of a log phase *E. coli* CN13 culture by inoculating media (e.g. 50 mL LB⁺N) with 600 µL overnight starter culture and incubation at 37-40 °C, 180 rpm until OD520 = 0.8;
d. Centrifugation of the log phase culture (3500 rpm, 5 min, 20 °C);
e. Resuspension of the cell pellet in LB⁺N: 3% gelatin solution (1: 1, e.g 25 mL); and
f. Aliquoting of the gelatin-bacteria suspension into a 1.5 mL screw cap tube with o-rings or 10 mL tube or 50 mL tube or 7 mL McCartney Bottle or 30 mL McCartney bottle, and storage at 4-8 °C for up to 2-6 months.

### Example 3: Quantitative enumeration of somatic coliphages (QP assay)

### 3. CFAP-based assay for quantitative enumeration of somatic coliphages in a 1 mL test sample using immobilised or freshly prepared early-mid log phase E. coli CN13 bacterial host cells and detection of bacterial β-galactosidase using CPRG:

a. Preparation of serial dilutions of the 1 mL test sample using LB medium (amended with nalidixic acid, LB⁺N);
b. The gel/immobilised cells is allowed to come to room temperature (~25 °C) for ~5 min and reverts to a liquid. For each assay, 450 µL LB⁺N is added to 150 µL cells, and the suspension is incubated at 37 °C for 40 min; tubes are placed in a horizontal position and incubated without shaking, and mixed (by inversion) every 15 min. *Cells and media for multiple assays can be combined for this incubation (e.g. for 10 assays, 4.5 mL LB⁺N* + *1.5 mL cells can be added to a 15 mL tube).* Alternatively, 600 µL of freshly prepared early-mid log phase culture may be used instead of immobilised cells (e.g. early-mid log phase culture: 25 mL LB+N is inoculated with 600 µL of an overnight starter culture and incubated at 37-40 °C, 180-200 rpm ~ 1hour until OD520 ~ 0.3-0.4; 50 µL 0.1M IPTG may be included in this early-mid log phase culture instead of including at step 3b);
c. Substrate and inducing agent are then added: for one assay 8 µL 25 mM CPRG and 4.8 µL 0.1 M IPTG are added to 600 µL cell suspension *(for 10 assays, 80 µL 25 mM CPRG and 48 µL 0.1 MIPTG are added to 6 mL cells);*
d. The cell suspension (600 µL) is then added to 1 mL of the test sample/dilutions and the mixture (1.6 mL) is applied to 47 mm Advantec^{®} CFAP supplied in Petrie dishes or circles cut from Millipore CFAP (#CO83, sterilised by autoclaving, dried and placed in a sterile Petri dish). A negative control may consist of all reagents and bacterial cells with the addition 1 mL LB media or sterile water in place of the 1 mL test sample/dilutions. A further negative control may consist of all reagents (including the test sample/dilutions) with the addition of 600 µL LB media in place of the cell suspension.
e. The CFAP is incubated at 37-40 °C for 1.5 - 2 hours and up to 4.5 hours and then zones of colour change are observed/counted;
f. QP assay is completed in 1.5-2 hours for somatic coliphages which produce large plaques (high numbers of somatic coliphages (> 100 - 10⁵ PFU) are detected after 1.5 hours and low numbers of somatic coliphages (1 PFU) are detected after 2 hours) and up to 4-4.5 hours may be required for somatic coliphages which produce small plaques. Lower number of coliphages produce localised regions of colour change equivalent to "plaques" and higher numbers of coliphages produce colour change across the surface of the CFAP). The method enables 1 PFU/ml to be detected where 1 mL of test sample is analysed and provides equivalent results to the conventional double agar layer plaque assay. The time of the assay may be shortened even further by use of alternative culture media or inclusion of other additives in the culture media/assay and/or use of imaging technology.

The QP assay (for detection of somatic coliphages in 1 mL test samples) as described above was used for enumeration of somatic coliphage isolate φX174 and results were compared to the double agar layer (DAL) plaque assay (US-EPA Method 1602: Male-specific (F+) and somatic coliphage in water by single agar layer (SAL) procedure). As shown in Table 1 and Figure 4a, equivalent numbers of φX174 were determined using the QP assay performed with freshly prepared log phase bacterial cells, the QP assay performed with immobilised bacterial cells (prepared as described in Example 1) and the DAL.

The QP assay (for detection of somatic coliphages in 1 mL test samples) as described above was used for enumeration of somatic coliphages in wastewater sample viral concentrates and results were compared to the double agar layer (DAL) plaque assay (US-EPA Method 1602: Male-specific (F+) and somatic coliphage in water by single agar layer (SAL) procedure). As shown in Table 2, equivalent numbers of somatic coliphages were determined using the QP assay (performed with immobilised bacterial cells prepared as described in Example 1) and the DAL. The analysis of 1 mL polyethylene glycol (PEG) viral concentrates in the i-QP assay (LOD 1 PFU/mL) resulted in a theoretical overall method sensitivity of 22 PFU/L (where viruses were extracted from a 230 mL sample into 5 mL PEG concentrate; for analysis of 1 mL dilutions of viral concentrate in triplicate assays; when converted to numbers of coliphages present in the original samples; the overall method sensitivity of the QP assay where viral concentration is performed is dependent on how the concentration method is performed, such as volume of viral concentrate produced from a given sample volume).

### Example 4: Quantitative enumeration of F+ coliphage (QP assay)

### 4. CFAP-based assay for quantitative enumeration of F+ coliphages in a 1 mL test sample using freshly cultured E. coli 700891 bacterial host cells and detection of bacterial β-galactosidase using CPRG:

a. Preparation of serial dilutions of the test sample using LB medium (amended with ampicillin and streptomycin, AS).
b. Preparation of a starter bacterial culture by inoculation of 5 mL LB+AS with *E. coli* 700891 from a glycerol stock or plate and incubation at 37 °C, 200 rpm for 16-18 h.
c. Preparation of early log phase *E. coli* 700891 cultures by inoculation of 25 mL media (LB⁺AS + 50 µL 0.1 M IPTG) with 200 µl starter culture and incubation at 37-40 °C, 200 rpm (~60 min, OD520 = 0.1). Place cells on ice.
d. Combine 600 µL early log phase *E. coli* 700891 cells with 8 µL 25 mM CPRG + 1 mL test sample/dilutions (Tween 20 or Tween 80 may also be added to the suspension, for example to produce a final concentration of 0.05-0.5%). Incubation of the suspension at 37-40 °C for 15 min. Application of the suspension to 47 mm Advantec^{®} CFAP supplied in Petrie dishes and incubation at 37-40 °C for 2.5- 3 hours and then zones of colour change are observed/counted.
e. QP assay is completed in 2.5-3.5 hours for F+ coliphages: high numbers of F+ coliphages (> 100 - 10⁵ PFU) are detected after 2.5 hours and low numbers of F+ coliphages (1 PFU) are detected after 3-3.5 hours. The method enables 1 PFU/ml to be detected, where 1 mL of test sample is analysed, and provides equivalent results to the conventional double agar layer plaque assay. The time of the assay may be shortened even further by use of alternative culture media or inclusion of other additives in the culture media/assay and/or use of imaging technology.

The QP assay (for detection of F+ coliphages in 1 mL test samples) as described above was used for enumeration of F+ coliphage isolate MS2 and results were compared to the double agar layer (DAL) plaque assay (US-EPA Method 1602: Male-specific (F+) and somatic coliphage in water by single agar layer (SAL) procedure). As shown in Table 3, equivalent numbers of MS2 were determined using the QP assay performed with freshly prepared log phase bacterial cells and the DAL.

### Example 5: Quantitative enumeration of somatic coliphages (QP assay)

### 5. CFAP-based assay for quantitative enumeration of somatic coliphages in a 10-100 mL test sample using freshly cultured or immobilised E. coli CN13 bacterial host cells and detection of bacterial β-galactosidase using CPRG:

a. Preparation of an early-mid log phase *E. coli* CN13 culture by inoculating media (30 mL LB⁺N) with 600 µL - 1000 µL overnight starter culture and incubation at 37-40 °C, 180 rpm until OD520 = 1.0 (~60 - 90 min); Or activation of immobilised cells by adding an equal volume of LB+N media and incubating at 37-40 °C for 60 min (e.g. adding 7.5 mL media to 7.5 mL cells; immobilised cells are prepared as described in Example 2, using early-mid log phase cells cultured until OD520 ~ 0.8 and resuspended in half volume LB⁺N:3% gelatin solution 1:1);
b. Combining 750 µL of bacterial cells (prepared as described in 5a above) with 34 µL 0.1M IPTG, 40-50 µL 25 mM CPRG, 100 µL 10 mg/mL nalidixic acid, 400 µL 10 x LB⁺N and 10mL test sample; and applying this suspension to sterile 11.5 x 11.5 cm CFAP (such as Millipore CFAP #CO8) in a square Petri dish or plastic zip lock bag (e.g. 30 µM thickness). The sample may be pre-treated, for example by passing through a 40 - 70 µM mesh filter to remove particulates and/or a 0.2 -0.45 µM membrane to remove undesired organisms. A negative control may consist of all reagents and bacterial cells with the addition 10 mL sterile water in place of the 10 mL test sample. A further negative control may consist of all reagents (including the test sample/dilutions) with the addition of 750 µL LB media or sterile water in place of the cell suspension.
c. Incubation of the inoculated CFAP at 37-40 °C for 2.5-4.5 h, and recording of colour change to determine coliphage presence (e.g. counting localised regions of colour change equivalent to "plaques" and/or observing colour change across the surface of the CFAP). The method enables 1 PFU/10 mL to be detected, where 10 mL of test sample is analysed, and provides quivalent results to the conventional single agar layer plaque assay. The limit of quantification is approximately 100 PFU/10 mL for undiluted samples; thus for samples with a higher number of coliphages dilution is required for quantitation. The time of the assay may be shortened even further by use of alternative culture media or inclusion of other additives in the culture media/assay and/or use of imaging technology;
d. For analysis of 100 mL samples, the reagents are multiplied by a factor of X10 and applied to 10 x (11.5 cm x 11.5 cm) sterile CFAP; Stock solutions of the combined reagents may be prepared so that only one solution needs to be applied in step 5b; The limit of quantification is approximately 1000 PFU/100 mL for undiluted samples; thus for samples with a higher number of coliphages dilution is required for quantitation.
e. Equivalent surface area CFAP of different shapes may be used (e.g. 10 x 15 cm rectangle CFAP X 9 may be used, where the assay mixture applied to each CFAP may consist of 11.1 mL test sample, 37.7 µL 0.1M IPTG, 44 µL 25 mM CPRG, 111.1 µL 10 mg/mL nalidixic acid, 410 µL 10 x LB⁺N and 1 mL bacterial cells.

The QP assay (for detection of somatic coliphages in 10 mL test samples) as described above was used for enumeration of somatic coliphage isolate φX174 and results were compared to the single agar layer (SAL) plaque assay (US-EPA Method 1602: Male-specific (F+) and somatic coliphage in water by single agar layer (SAL) procedure). As shown in Table 4 and Figure 4b, equivalent numbers of φX174 were determined using the QP performed with freshly prepared log phase bacterial cells, the QP assay performed with immobilised bacterial cells (prepared as described in Example 2) and the SAL.

The QP assay (for detection of somatic coliphages in 10 mL test samples) as described above was used for enumeration of somatic coliphages in raw (unprocessed) freshwater lake samples and results were compared to the single agar layer (SAL) plaque assay (US-EPA Method 1602: Male-specific (F+) and somatic coliphage in water by single agar layer (SAL) procedure). As shown in Table 5, equivalent numbers of somatic coliphages were determined using the QP assay (performed with immobilised bacterial cells prepared as described in Example 2) and the SAL.

### Example 6: Quantitative enumeration of F+ coliphages (QP assay)

### 6. CFAP-based assay for quantitative enumeration of F+ coliphages in a 10-100 mL test sample using freshly cultured E. coli 700891 bacterial host cells and detection of bacterial β-galactosidase using CPRG:

a. Preparation of an early log phase *E. coli* 700891 culture by inoculating media (30 mL LB⁺AS + 50 µL 0.1 M IPTG) with 200 µL overnight starter culture and incubation at 37-40 °C, 200 rpm until OD520 = 0.2 (~60 - 90 min);
b. Combining 2.4 mL of bacterial cells (prepared as described in 6a above) with40 µL 25 mM CPRG, 100 µL 1.5 mg/mL streptomycin/ampicillin, 400 µL 10 x LB⁺N and 10mL test sample. The sample may be pre-treated, for example by passing through a 40 - 70 µM mesh filter to remove particulates and/or a 0.2 -0.45 µM membrane to remove undesired organisms;
c. Incubation of the suspension at 37-40 °C for 10 minutes; and addition of 70 µL 25% Tween 80 (and/or polyvinylpyrrolidone and/or Ficoll);
d. Applying the assay suspension to sterile 10 cm x 15 cm CFAP (such as Millipore CFAP #CO8) in a plastic zip lock bag (e.g. 30 µM thickness);
e. Incubation of the inoculated CFAP at 37-40 °C for 2.5-4.5 h, and recording of colour change to determine coliphage presence (e.g. counting localised regions of colour change equivalent to "plaques" and/or observing colour change across the surface of the CFAP). The method enables 1 PFU/10 mL to be detected, where 10 mL of test sample is analysed, and provides equivalent results to the conventional single agar layer plaque assay. The time of the assay may be shortened even further by use of alternative culture media or inclusion of other additives in the culture media/assay and/or use of imaging technology;
f. For analysis of 100 mL samples, the reagents are multiplied by a factor of X10 and applied to 10 X (10 cm x 15 cm) sterile CFAP; Stock solutions of the combined reagents may be prepared so that only one solution needs to be applied in step 6b.
g. For determination of counts of F+RNA and F+DNA coliphages, an additional test sample may be analysed as above with RNAse added to the assay suspension (e.g. 30 µg/mL) to inhibit the growth of F+RNA coliphages and thus F+DNA coliphages will be enumerated; F+RNA coliphage counts can then be determined by subtracting the F+DNA coliphage count from the total count determined in test sample assayed without addition of RNase.

### Example 7: lyophilisation for preservation of bacterial host cells

### 7. Use of lyophilisation for preservation of bacterial host cells, including methods to support rapid cell recovery for use in QP assays that include β-galactosidase detection:

a. Preparation of a starter bacterial culture by inoculating 5 mL LB+ (amended with nalidixic acid, LB+N) with *E. coli* CN13 from a glycerol stock or agar plate and incubation at 37 °C, 180 rpm for 16-18 h;
b. Preparation of a log phase *E. coli* CN13 culture by inoculating media (60 mL LB⁺N) with 600 µL starter culture and incubation at 37-40 °C, 180 rpm until OD520 = 0.8;
c. Centrifugation of the log phase culture (3500 rpm, 5 min, 20 °C);
d. Resuspension of the cell pellet in 1/20 volume of cryo-solution (e.g. 60 mL bacterial culture is resuspended in 3 mL cryo-solution), where the cryo- solution for example may consist of combinations of reagents such as:
   i. 10% peptone + 10% trehalose (± 10 % raffinose)
   ii. 10% peptone + 5% trehalose (± 10 % raffinose)
   iii. 10% tryptone + 10% trehalose (± 10 % raffinose)
h. The cell suspension may be frozen and lyophilised or the cell suspension may be applied to sterilised cellulose fibre sample pad prior to freezing and lyophilisation such as:
   i. Millipore C248 CFAP (1 x 2.5 cm) in 5 ml vacule vial (more than one strip of C248 may be inoculated per vial);
   ii. Millipore CFAP #CO83 (50 mm diameter circles or 11.5 x 11.5 cm squares using optimised cell numbers);
i. Freezing cells at -70 °C or below for at least 1 hour;
j. Lyophilisation of the cells overnight (e.g. 500 µBar, 16-18 h) using a freeze dryer and sealing of vials/containers under vacuum;
k. Storage of lyophilised cultures at 4 °C;
l. Reactivation of cells by adding bacterial culture media (such as LB+ media without antibiotics ± 0.01 to 0.1% cas-amino acids) and incubating without shaking at 40 °C for 1 - 2 hour;
m. The re-activated cells may be used in assays for coliphage detection as described in 5b (750 µL cells for the 10 mL assay) and 3b (600 µL cells for the 1 mL assay).

### Example 8: Confirmation of the QuantiPhage Assay

### 8. Confirmation that QuantiPhage Assay signals are due to coliphages using spot plates (somatic coliphage method):

a. Extract CFAP from a localised region of colour change/plaques using a sterile scalpel blade;
b. Immerse the CFAP in 150 µL LB⁺N in a microfuge tube for 5 min and vortex
c. Pipette 5 µL of the suspension onto spot plates (prepared as described in US-EPA Method 1602: Male-specific (F+) and somatic coliphage in water by single agar layer (SAL) procedure, 2001) and;
d. Incubate plates for 16-18 h at 37 °C and observe cleared zones due to passaged coliphages.

Throughout the specification the aim has been to describe the preferred embodiments of the invention without limiting the invention to any one embodiment or specific collection of features.

### TABLES

**Table 1. Detection of somatic coliphage isolate φX174 using the QP assay (1 mL) with log phase and immobilised cells.**

| **Assay format a** | **Spike b (PFU)** | **Number of Replicates** | **Mean number of coliphages (PFU/mL)** |
|---|---|---|---|
| DAL | 1 | 10 | 1.0 ± 0.7 |
| L-QP | 1 | 10 | 1.4 ± 1.3 |
| i-QP | 1 | 10 | 1.1 ± 0.8 |
| DAL | 10 | 3 | 8.7 ± 4.2 |
| L-QP | 10 | 3 | 13.3 ± 1.5 |
| i-QP | 10 | 3 | 10.0 ± 1.7 |

| | | | |
|---|---|---|---|
| a. DAL = double agar layer plaque assay; L-QP = QP assay (1 mL) using freshly prepared log phase bacterial cells; i-QP = QP assay (1 mL) using gelatin-immobilised bacterial cells. b. LB media was spiked with somatic coliphage isolate φX174. | | | |

**Table 2. Detection of somatic coliphages in wastewater samples (1 mL viral concentrates) using the QP assay (1 mL).**

| **Assay format a** | **Number of Replicates** | **Water matrix b** | **Mean number of coliphages (log10 PFU/L)** |
|---|---|---|---|
| DAL | 14 | WSP-I | 4.65 ± 0.25 |
| i-QP | 14 | WSP-I | 4.64 ± 0.30 |
| DAL | 6 | WSP-O | 0.41 ± 0.69 |
| i-QP | 6 | WSP-O | 0.26 ± 0.64 |

| | | | |
|---|---|---|---|
| a. DAL = double agar layer plaque assay (US-EPA Method 1602); i-QP = QP assay (1 mL) using gelatin-immobilised bacterial cells; means determined from three technical replicates per each replicate sample. b. WSP-I = waste stabilisation pond inlet sample, viral concentrate prepared by polyethylene glycol precipitation and chloroform extraction of 230 mL sample; WSP-O = waste stabilisation pond outlet sample, prepared as for WSP-I. | | | |

**Table 3. Detection of F+ coliphage isolate MS2 using the QP assay (1 mL).**

| **Assay format** | **Spike (PFU)** | **Number of Replicates** | **Mean number of coliphages (PFU/mL)** |
|---|---|---|---|
| DAL | 1 | 3 | 2.0 ± 1.0 |
| L-QP | 1 | 3 | 2.3 ± 1.5 |
| DAL | 10 | 3 | 8.0 ± 1.7 |
| L-QP | 10 | 3 | 7.0 ± 1.0 |

| | | | |
|---|---|---|---|
| a. DAL = double agar layer plaque assay; L-QP = QP assay (1 mL) using freshly prepared log phase bacterial cells. b. LB broth was spiked with F+ coliphage isolate MS2. | | | |

**Table 4. Detection of somatic coliphage isolate φX174 using the QP assay (10 mL) with log phase and immobilised bacterial cells.**

| **Assay format a** | **Spike b (PFU)** | **Number of Replicates** | **Mean number of coliphages (PFU/10 mL)** |
|---|---|---|---|
| SAL | 1 | 10 | 1.0 ± 0.8 |
| L-QP | 1 | 10 | 1.0 ± 1.1 |
| i-QP | 1 | 10 | 1.2 ± 0.6 |
| SAL | 10 | 3 | 10.7 ± 1.5 |
| L-QP | 10 | 3 | 11.0 ± 3.6 |
| i-QP | 10 | 3 | 10.3 ± 4.0 |

| | | | |
|---|---|---|---|
| a. SAL = single agar layer plaque assay; L-QP = QP assay (10 mL) using freshly prepared log phase bacterial cells; i-QP = QP assay (10 mL) using gelatin-immobilised bacterial cells b. Sterile distilled water was spiked with somatic coliphage isolate φX174. | | | |

**Table 5. Detection of somatic coliphages in raw freshwater lake samples using the QP assay (10 mL).**

| **Assay format** | **Sample No.** | **Spike (PFU)** | **Number of Replicates** | **Mean number of coliphages (PFU/10 mL)** |
|---|---|---|---|---|
| SAL | 1 | 10 | 10 | 94±49 |
| i-QP | 1 | 10 | 10 | 10.5 ± 2.9 |
| SAL | 1 | 0 | 10 | 0.3 ± 0.7 |
| i-QP | 1 | 0 | 10 | 0.8 ± 0.9 |
| SAL | 2 | 0 | 10 | 0.0 ± 0.0 |
| i-QP | 2 | 0 | 10 | 0.5 ± 1.0 |
| SAL | 3 | 0 | 10 | 0.1 ± 0.3 |
| i-QP | 3 | 0 | 10 | 0.6 ± 1.1 |

| | | | | |
|---|---|---|---|---|
| a. SAL = single agar layer plaque assay; i-QP = QP assay using gelatin-immobilised bacterial cells b. The sample was spiked with somatic coliphage isolate φX174 where indicated . | | | | |

## Claims

1. A method for culture and detection of a bacteriophage in a test sample, which includes the step of culturing the bacteriophage using a bacterium that is a host for the bacteriophage within an absorptive fibrous matrix; and quantitatively enumerating the bacteriophage by detecting discrete zones of change in a detectable signal within the absorptive fibrous matrix facilitated by a detection reagent that is a substrate molecule and is selected from the group consisting of a chromogenic, colorimetric, fluorescent, electrochemical, bioluminescent, photoluminescent, and chemiluminescent detection reagent, preferably wherein the bacteriophage is detected subsequent to lysis of bacterial cells of the bacterium.

2. The method of Claim 1, wherein the change in the detectable signal is a colour change.

3. The method of Claim 1 or Claim 2, wherein the absorptive fibrous matrix is or comprises a cellulosic material, preferably wherein the absorptive fibrous matrix is a cellulose fibre absorbent pad (CFAP).

4. The method of any preceding claim, wherein prior to culturing and detecting the bacteriophage, the test sample is combined with bacterial cells of the bacterium that is a host for the bacteriophage, preferably wherein the bacterial cells are early-mid log phase cultures, reactivated immobilised cells or otherwise bacterial cells that exhibit low rates of inherent and/or non-specific cell lysis.

5. The method of Claim 3, wherein the test sample, bacteria, a bacterial culture medium, and/or the detection reagent are contacted with the absorptive fibrous matrix to thereby impregnate or otherwise locate the test sample, bacteria, bacterial culture media and/or the detection reagent within the absorptive fibrous matrix.

6. The method of Claim 5, wherein the absorptive fibrous matrix is incubated under conditions of time and temperature appropriate for bacteriophage replication and detection, wherein low rates of inherent and/or non-specific bacterial cell lysis are maintained.

7. The method of Claim 5 or Claim 6, wherein the test sample is applied to substantially the entire surface of the absorptive fibrous matrix to thereby substantially or completely saturate the absorptive fibrous matrix.

8. The method of any one of Claims 4-7, including the step of inducing expression of one or more genes by the bacterial host cells, wherein the detection reagent is capable of eliciting the detectable signal upon detecting the expression of the one or more genes, their encoded protein(s) or a metabolic or cellular product thereof.

9. The method of Claim 8, wherein the presence of bacteriophage results in the detection reagent eliciting a signal upon detecting the expression of the one or more genes, their encoded protein(s) or metabolic or cellular product thereof.

10. The method of any preceding claim, wherein the bacteriophage are coliphage, preferably wherein the coliphage are somatic coliphage, F+ RNA coliphage and/or F+ DNA coliphage.

11. A method for culture and detection of a bacteriophage in a test sample, which includes the step of culturing and detecting the bacteriophage within an absorptive fibrous matrix, wherein:
i. prior to culturing and detecting the bacteriophage, the test sample is combined with bacterial cells of a bacterium that is a host for the bacteriophage, wherein the bacterial cells are early-mid log phase cultures, reactivated immobilised cells or otherwise exhibit low rates of inherent and/or non-specific cell lysis;
ii. a detection reagent that is a substrate molecule and is selected from the group consisting of a chromogenic, colorimetric, fluorescent, electrochemical, bioluminescent, photoluminescent, and chemiluminescent detection reagent is added to the test sample comprising the bacterial host cells;
iii. a combination of the test sample and the bacteria, a bacterial culture medium and the detection reagent is applied to substantially the entire surface of the absorptive fibrous matrix to thereby substantially or completely saturate the absorptive fibrous matrix to facilitate localisation of bacterial cells within the absorptive fibrous matrix;
iv. the absorptive fibrous matrix is incubated under conditions of time and temperature appropriate for bacteriophage replication and detection, wherein low rates of inherent and/or non-specific bacterial cell lysis are maintained; and
v. localised, discrete regions of change in a detectable signal within the absorptive fibrous matrix facilitated by the detection reagent can be detected, to thereby quantitatively enumerate the bacteriophage in the test sample.

12. The method of Claim 11, wherein the change in the detectable signal is a colour change.

13. A method for culture and detection of a bacteriophage in a test sample for quantitative enumeration of bacteriophages, which includes the steps of:
i. combining the test sample with bacterial host cells that are early-mid log phase cultures, reactivated immobilised cells or otherwise have low rates of inherent and/or non-specific bacterial lysis to produce an assay suspension;
ii. providing a detection reagent that is, or comprises, a chromogen/colorimetric or fluorescent substrate that can be hydrolysed/acted on by a bacterial enzyme or reacts with/binds to a bacterial metabolic or cellular product that is released from the bacterial cell following bacteriophage-induced lysis;
iii. providing an inducing agent to induce expression of the assayed bacterial enzyme or metabolic/cellular products;
iv. applying the assay suspension to an absorptive fibrous matrix such as a CFAP, where the assay suspension is applied to the entire surface of the absorptive fibrous matrix, resulting in saturation or sub-saturation of the matrix;
v. incubation of the absorptive fibrous matrix under suitable conditions of time and temperature; and
vi. detection of the presence of bacteriophage by observing a colour change on the surface of the absorptive fibrous matrix and quantitative enumeration thereof or detection of an absence of bacteriophage by the absence of a colour change.

14. The method of any preceding claim, wherein the test sample is, or comprises: water, inclusive of drinking water, groundwater, wastewater, wastewater effluent, recycled water, combined sewer overflows, recreational water, surface water,
river water, sea water, lake water, irrigation water, swimming pool water, spa water, greywater and septic system water; other wastewater treatment by-products such as sludge and compost; food and beverage products, plant or animal tissue/material/samples; clinical isolates or samples inclusive of samples from diseased plants or animals; and/or laboratory isolates or samples.

15. Use of a kit for culture and detection of a bacteriophage in a test sample according to the method of any preceding claim, the kit comprising: an absorptive fibrous matrix for use in directly supporting growth and detection of bacteriophage, preferably wherein the absorptive fibrous matrix is a cellulosic material, more preferably wherein the absorptive fibrous matrix is a cellulose fibre absorbent pad (CFAP); a detection reagent that is a substrate molecule and is selected from the group consisting of a chromogenic, colorimetric, fluorescent, electrochemical, bioluminescent, photoluminescent, and chemiluminescent detection reagent; and, optionally, one or more additional components selected from the group consisting of (a) a culture medium or one or more components thereof; (b) an inducing agent; (c) bacterial cells of a bacterium that is a host for the bacteriophage, preferably wherein the bacterial cells are lyophilised, dried, or thermo-reversibly immobilised, wherein the bacterial cells are separate to or within or on the absorptive fibrous matrix; (d) a medium for preserving or immobilising bacterial cells, preferably wherein the medium is for thermo-reversible immobilisation of bacterial cells, preferably wherein the medium comprises gelatin; (e) RNAse; (f) positive control bacteriophage; and/or (g) one or more vessels, containers or enclosures capable of housing the absorptive fibrous matrix when in use.

## Patentansprüche

1. Verfahren zur Kultivierung und zum Nachweis eines Bakteriophagen in einer Testprobe, das den Schritt des Kultivierens des Bakteriophagen unter Verwendung eines Bakteriums, das ein Wirt für den Bakteriophagen ist, innerhalb einer absorbierenden faserigen Matrix einschließt; und quantitatives Auszählen des Bakteriophagen durch Nachweisen diskreter Zonen von Veränderung in einem nachweisbaren Signal innerhalb der absorbierenden faserigen Matrix, unterstützt durch ein Nachweisreagenz, das ein Substratmolekül ist und ausgewählt ist aus der Gruppe, bestehend aus einem chromogenen, kolorimetrischen, fluoreszierenden, elektrochemischen, biolumineszenten, photolumineszenten, und chemilumineszenten Nachweisreagenz, vorzugsweise wobei der Bakteriophage nach der Lyse von Bakterienzellen des Bakteriums nachgewiesen wird.

2. Verfahren nach Anspruch 1, wobei die Veränderung des nachweisbaren Signals eine Farbänderung ist.

3. Verfahren nach Anspruch 1 oder Anspruch 2, wobei die absorbierende faserige Matrix ein zellulosehaltiges Material ist oder umfasst, vorzugsweise wobei die absorbierende faserige Matrix ein Zellulosefaser-Absorptionskissen (CFAP) ist.

4. Verfahren nach einem der vorhergehenden Ansprüche, wobei vor Kultivieren und Nachweisen des Bakteriophagen die Testprobe mit Bakterienzellen des Bakteriums, das ein Wirt für den Bakteriophagen ist, kombiniert wird, vorzugsweise wobei die Bakterienzellen frühe bis mittleren log-Phase Kulturen, reaktivierte immobilisierte Zellen oder andere Bakterienzellen sind, die geringe Raten inhärenter und/oder unspezifischer Zelllyse aufweisen.

5. Verfahren nach Anspruch 3, wobei die Testprobe, die Bakterien, ein bakterielles Kulturmedium und/oder das Nachweisreagenz mit der absorbierenden faserigen Matrix in Kontakt gebracht werden, um dadurch die Testprobe, die Bakterien, das bakterielle Kulturmedium und/oder das Nachweisreagenz innerhalb der absorbierenden faserigen Matrix zu imprägnieren oder anderweitig zu lokalisieren.

6. Verfahren nach Anspruch 5, wobei die absorbierende faserige Matrix unter Zeit- und Temperaturbedingungen inkubiert wird, geeignet für Bakteriophagen Replikation und Nachweis, wobei niedrige Raten von inhärenter und/oder unspezifischer bakterieller Zelllyse aufrechterhalten werden.

7. Verfahren nach Anspruch 5 oder Anspruch 6, wobei die Testprobe im Wesentlichen auf die gesamte Oberfläche der absorbierenden faserigen Matrix aufgetragen wird, um dadurch die absorbierende faserige Matrix im Wesentlichen oder vollständig zu sättigen.

8. Verfahren nach einem der Ansprüche 4 - 7, einschließend den Schritt von Induzieren von Expression eines oder mehrerer Gene durch die bakteriellen Wirtszellen, wobei das Nachweisreagenz, fähig ist, zum Auslösen des nachweisbaren Signals beim Nachweisen der Expression des einen oder der mehreren Gene, ihres kodierten Proteins (ihrer kodierten Proteine) oder eines metabolischen oder zellulären Produkts davon.

9. Verfahren nach Anspruch 8, wobei das Vorhandensein von Bakteriophagen in dem Auslösen des Nachweisreagenzes eines Signals, beim Nachweisen der Expression eines oder mehrerer Gene, ihres kodierten Proteins (ihrer kodierten Proteine) oder deren metabolisches oder zelluläres Produkt.

10. Verfahren nach einem der vorhergehenden Ansprüche, wobei die Bakteriophagen Coliphagen sind, vorzugsweise wobei die Coliphagensomatische Coliphagen, F+ RNA Coliphagen und/oder F+ DNA Coliphagen sind.

11. Verfahren zur Kultivierung und zum Nachweis eines Bakteriophagen in einer Testprobe, das den Schritt des Kultivierens und Nachweisens des Bakteriophagen innerhalb einer absorbierenden faserigen Matrix einschließt, wobei:
i. vor Kultivieren und Nachweisen des Bakteriophagen die Testprobe mit Bakterienzellen eines Bakteriums kombiniert wird, das ein Wirt für den Bakteriophagen ist, wobei die Bakterienzellen frühe bis mittlere log-Phase Kulturen, reaktivierte immobilisierte Zellen sind oder anderweitig geringe Raten inhärenter und/oder unspezifischer Zelllyse aufweisen;
ii. ein Nachweisreagenz, das ein Substratmolekül ist und ausgewählt ist aus der Gruppe, bestehend aus einem chromogenen, kolorimetrischen, fluoreszierenden, elektrochemischen, biolumineszenten, photolumineszenten und chemilumineszenten Nachweisreagenz, zu der Testprobe, umfassend die bakteriellen Wirtszellen, zugegeben wird;
iii. eine Kombination der Testprobe und den Bakterien, einem bakteriellen Kulturmedium und dem Nachweisreagenz auf im Wesentlichen die gesamte Oberfläche der absorbierenden faserigen Matrix aufgebracht wird, um dadurch die absorbierende faserige Matrix im Wesentlichen oder vollständig zu sättigen, um Lokalisierung der bakteriellen Zellen innerhalb der absorbierenden faserigen Matrix zu unterstützen;
iv. die absorbierende faserige Matrix unter Zeit- und Temperaturbedingungen inkubiert wird, geeignet für Bakteriophagen Replikation und Nachweis, wobei niedrige Raten von inhärenter und/oder unspezifischer bakterieller Zelllyse aufrechterhalten werden; und
v. lokalisierte, diskrete Bereiche von Veränderung in einem nachweisbaren Signal innerhalb der absorbierenden faserigen Matrix, unterstützt durch das Nachweisreagenz, nachgewiesen werden können, um dadurch die Bakteriophagen in der Testprobe quantitativ zu zählen.

12. Verfahren nach Anspruch 11, wobei die Veränderung des nachweisbaren Signals eine Farbveränderung ist.

13. Verfahren zur Kultivierung und zum Nachweis eines Bakteriophagen in einer Testprobe zur quantitativen Auszählung von Bakteriophagen, das die Schritte einschließt von:
i. Kombinieren der Testprobe mit bakteriellen Wirtszellen, die frühe bis mittlere log-Phase Kulturen, reaktivierte immobilisierte Zellen sind oder anderweitig niedrige Raten inhärenter und/oder unspezifischer bakterieller Lyse haben, um eine Assay-Suspension herzustellen;
ii. Bereitstellen eines Nachweisreagenzes, das ein chromogenes/kolorimetrisches oder fluoreszierendes Substrat ist oder umfasst, das von einem bakteriellen Enzym hydrolysiert/umgesetzt werden kann oder mit einem bakteriellen metabolischen oder zellulären Produkt, das von der Bakterienzelle freigesetzt wird, folgend einer Bakteriophagen- induzierten Lyse, reagiert/an dieses bindet;
iii. Bereitstellen eines induzierenden Mittels, um Expression des untersuchten bakteriellen Enzyms oder der metabolischen/zellulären Produkte zu induzieren;
iv. Auftragen der Assay-Suspension auf eine absorbierende faserige Matrix wie eine CFAP, wobei die Assay-Suspension auf die gesamte Oberfläche der absorbierenden faserigen Matrix aufgetragen wird, resultierend in Sättigung oder Untersättigung der Matrix;
v. Inkubation der absorbierenden faserigen Matrix unter passenden Zeit- und Temperaturbedingungen; und
vi. Nachweis des Vorhandenseins von Bakteriophagen durch Beobachten einer Farbveränderung auf der Oberfläche der absorbierenden faserigen Matrix und quantitativer Auszählung davon oder Nachweis der Abwesenheit von Bakteriophagen durch das Fehlen einer Farbveränderung.

14. Methode nach einem der vorhergehenden Ansprüche, wobei die Testprobe Wasser ist oder umfasst, einschließlich Trinkwasser, Grundwasser, Abwasser, Abwasserabfluss, recyceltes Wasser, kombinierte Abwasserüberläufe, Wasseraufbereitung, Oberflächenwasser,
Flusswasser, Meerwasser, Seewasser, Bewässerungswasser, Schwimmbadwasser, Spa-Wasser, Grauwasser und Wasser aus Kläranlagen; andere Nebenprodukte der Abwasserbehandlung wie Schlamm und Kompost; Lebensmittel- und Getränkeprodukte, pflanzliches oder tierisches Gewebe/Material/Proben; klinische Isolate oder Proben, einschließlich Proben von kranken Pflanzen oder Tieren; und/oder Laborisolate oder Proben.

15. Verwendung eines Kits zur Kultivierung und zum Nachweis eines Bakteriophagen in einer Testprobe nach dem Verfahren nach einem der vorhergehenden Ansprüche, das Kit umfassend: eine absorbierende faserige Matrix zur Verwendung beim direkten Unterstützen von Wachstum und Nachweis von Bakteriophagen, vorzugsweise wobei die absorbierende faserige Matrix ein zellulosehaltiges Material ist, bevorzugter wobei die absorbierende faserige Matrix ein Zellulosefaser-Absorptionskissen (CFAP) ist; ein Nachweisreagenz, das ein Substratmolekül ist und ausgewählt ist aus der Gruppe, bestehend aus einem chromogenen, kolorimetrischen, fluoreszierenden, elektrochemischen, biolumineszenten, photolumineszenten und chemilumineszenten Nachweisreagenz; und optional eine oder mehrere zusätzliche Komponenten, ausgewählt aus der Gruppe, bestehend aus (a) einem Kulturmedium oder einer oder mehreren Komponenten davon; (b) einem induzierenden Mittel; (c) Bakterienzellen eines Bakteriums, das ein Wirt für den Bakteriophagen ist, vorzugsweise wobei die Bakterienzellenlyophilisiert, getrocknet oder thermoreversibel immobilisiert sind, wobei die Bakterienzellen getrennt von, in oder auf der absorbierenden faserigen Matrix sind; (d) ein Medium zum Konservieren oder Immobilisieren von Bakterienzellen, vorzugsweise wobei das Medium zur thermoreversiblen Immobilisierung von Bakterienzellen ist, vorzugsweise wobei das Medium Gelatine umfasst; (e) RNAse; (f) Positivkontrolle Bakteriophagen; und/oder (g) ein oder mehrere Gefäße, Behälter oder Gehäuse, fähig zum Aufnehmen der absorbierenden faserigen Matrix bei der Verwendung.

## Revendications

1. Un procédé de culture et de détection d'un bactériophage dans un échantillon test, qui inclut l'étape de culture du bactériophage en employant une bactérie qui est un hôte pour le bactériophage dans une matrice fibreuse absorbante; et l'énumération quantitative du bactériophage par détection des zones de changement discrètes dans un signal détectable dans la matrice fibreuse absorbante facilitée par un réactif de détection qui est une molécule de substrat et qui est choisi dans le groupe consistant en les réactifs de détection chromogénique, colorimétrique, fluorescente, électrochimique, bioluminescente, photoluminescente et chimioluminescente de préférence dans lequel le bactériophage est détecté subséquemment à la lyse des cellules bactériennes de la bactérie.

2. Le procédé selon la revendication 1, dans lequel le changement dans le signal détectable consiste en un changement de couleur.

3. Le procédé selon la revendication 1 ou la revendication 2, dans lequel la matrice fibreuse absorbante est, ou comprend, un matériau cellulosique, la matrice fibreuse absorbante étant de préférence un tampon absorbant de fibres cellulosiques (CFAP).

4. Le procédé selon l'une quelconque des revendications précédentes, dans lequel, préalablement à la culture et à la détection du bactériophage, l'échantillon test est combiné avec des cellules bactériennes de la bactérie qui est un hôte du bactériophage, de préférence les cellules bactériennes étant des cultures en phase logarithmique intermédiaire précoce, des cellules immobilisées réactivées ou d'autres cellules bactériennes présentant de faibles taux de lyse cellulaire inhérente et/ou non spécifique.

5. Le procédé selon la revendication 3, dans lequel l'échantillon test, les bactéries, un milieu de culture bactérienne et/ou le réactif de détection sont mis en contact avec la matrice fibreuse absorbante afin d'imprégner ou autrement localiser ainsi l'échantillon test, les bactéries, les milieux de culture bactérienne et/ou le réactif de détection dans la matrice fibreuse absorbante.

6. Le procédé selon la revendication 5, dans lequel la matrice fibreuse absorbante est incubée dans des conditions de temps et de température appropriés pour la réplication et la détection des bactériophages, dans lequel sont maintenus les faibles taux de lyse cellulaire bactérienne inhérente et/ou non spécifique.

7. Le procédé selon la revendication 5 ou la revendication 6, dans lequel l'échantillon test est appliqué sur la quasi-totalité de la surface de la matrice fibreuse absorbante pour saturer ainsi substantiellement ou complètement la matrice fibreuse absorbante.

8. Le procédé selon l'une quelconque des revendications 4 à 7, incluant l'étape consistant à induire l'expression d'un ou plusieurs gènes par les cellules hôtes bactériennes, dans lequel le réactif de détection est capable de déclencher le signal détectable lors de la détection de l'expression de l'un ou de plusieurs gènes, de leur(s) protéine(s) codée(s) ou d'un produit métabolique ou cellulaire en dérivant.

9. Le procédé selon la revendication 8, dans lequel la présence de bactériophage a pour conséquence le déclenchement dans le réactif de détection le déclenchement d'un signal lors de la détection de l'expression d'un ou plusieurs gènes, de leur(s) protéine(s) encodée(s) ou de leur produit métabolique ou cellulaire en dérivant.

10. Le procédé selon une quelconque des revendications précédentes, dans lequel les bactériophages sont des coliphages, de préférence les coliphages étant des coliphages somatiques, des coliphages à ARN F+ et/ou des coliphages d'ADN F+.

11. Un procédé de culture et de détection d'un bactériophage dans un échantillon test, qui inclut l'étape de culture et de détection du bactériophage dans une matrice fibreuse absorbante, dans laquelle:
i.. avant de cultiver et de détecter le bactériophage, l'échantillon test est combiné avec des cellules bactériennes d'une bactérie qui est un hôte du bactériophage, dans lequel les cellules bactériennes sont des cultures en phase logarithmique intermédiaire précoce, des cellules immobilisées réactivées ou autrement présentent de faibles taux de lyse cellulaire inhérente et/ou non spécifique;
ii. un réactif de détection qui est une molécule de substrat et qui est choisi dans le groupe consistant en les réactifs de détection chromogénique, colorimétrique, fluorescente, électrochimique, bioluminescente, photoluminescente et chimioluminescente est ajouté à l'échantillon test comprenant les cellules hôtes bactériennes;
iii. une combinaison de l'échantillon test et des bactéries, d'un milieu de culture bactérien et du réactif de détection est appliquée sur la quasi-totalité de la surface de la matrice fibreuse absorbante afin de saturer substantiellement ou complètement la matrice fibreuse absorbante pour faciliter la localisation des cellules bactériennes dans la matrice fibreuse absorbante;
iv. la matrice fibreuse absorbante est incubée dans des conditions de temps et de température appropriées pour permettre la réplication des bactériophages et leur détection, dans lequel sont maintenus de faibles taux de lyse cellulaire bactérienne inhérente et/ou non spécifique; et
v. des régions de changement localisées et discrètes dans un signal détectable dans la matrice fibreuse absorbante facilitée par le réactif de détection peuvent être détectées, ce qui permet d'énumérer quantitativement le bactériophage dans l' échantillon test.

12. Le procédé selon la revendication 11, dans lequel le changement du signal détectable est un changement de couleur.

13. Un procédé de culture et de détection d'un bactériophage dans un échantillon test pour l'énumération quantitative des bactériophages, qui inclut les étapes suivantes:
i. combiner l'échantillon test avec des cellules hôtes bactériennes qui sont des cultures en phase logarithmique précoce intermédiaire moyenne, des cellules immobilisées réactivées ou autrement présentent de faibles taux de lyse bactérienne inhérente et/ou non spécifique pour produire une suspension test;
ii. fournir un réactif de détection qui est, ou comprend, un substrat chromogène/colorimétrique ou fluorescent qui peut être hydrolysé/traité par une enzyme bactérienne ou qui réagit avec ou se lie à un produit métabolique ou cellulaire bactérien qui s'est libéré de la cellule bactérienne à la suite d'une lyse induite par les bactériophages;
iii. fournir un agent inducteur pour induire l'expression de l'enzyme bactérienne testée ou des produits métaboliques/cellulaires;
iv. appliquer la suspension test à une matrice fibreuse absorbante telle qu'un CFAP, lorsque la suspension test est appliquée sur toute la surface de la matrice fibreuse absorbante, entraînant une saturation ou une sous-saturation de la matrice;
v. incuber la matrice fibreuse absorbante dans des conditions de temps et de température appropriées; et
vi. détecter la présence de bactériophages par l'observation d'un changement de couleur à la surface de la matrice fibreuse absorbante et son énumération quantitative ou la détection d'une absence de bactériophage par l'absence de changement de couleur.

14. Le procédé selon une quelconque des revendications précédentes, , dans lequel l'échantillon test consiste ou comprend: l'eau, y compris l'eau potable, les eaux souterraines, les eaux usées, les effluents d'eaux usées, l'eau recyclée, les débordements d'égouts unitaires, les eaux récréatives, les eaux de surface, les eaux de rivière, l'eau de mer, l'eau de lac, l'eau d'irrigation, l'eau de piscine, l'eau de spa, les eaux grises et l'eau de fosse septique; d'autres sous-produits du traitement des eaux usées tels que les boues et le compost; les produits alimentaires et boissons, les tissus/matières/échantillons végétaux ou animaux; les déchets cliniques ou échantillons, y compris des échantillons de plantes ou d'animaux malades; et/ou des déchets ou des échantillons de laboratoire.

15. Utilisation d'un kit de culture et de détection d'un bactériophage dans un échantillon test obtenu selon le procédé de l'une quelconque des revendications précédentes, le kit comprenant : une matrice fibreuse absorbante destinée à être utilisée pour contribuer directement à la croissance et la détection du bactériophage, de préférence dans laquelle la matrice fibreuse absorbante est un matériau cellulosique, plus préférentiellement dans laquelle la matrice fibreuse absorbante est un tampon absorbant de fibres de cellulose (CFAP); un réactif de détection qui est une molécule de substrat et qui est choisi dans le groupe constitué par les réactifs de détection chromogénique, colorimétrique, fluorescente électrochimique, bioluminescente, photoluminescente et chimioluminescente; et, éventuellement, un ou plusieurs composants supplémentaires choisis dans le groupe constitué
a) d'un milieu de culture ou d'un ou plusieurs de ses composants en dérivant;
b) d'un agent inducteur;
c) de cellules bactériennes d'une bactérie hôte du bactériophage, de préférence lesquelles cellules bactériennes sont lyophilisées, séchées ou thermo-immobilisées de manière réversible, lesquelles cellules bactériennes sont séparées de la matrice fibreuse absorbante ou contenues à l'intérieur de celle-ci ou étalées sur celle-ci;
d) un milieu de conservation ou d'immobilisation de cellules bactériennes, de préférence le milieu étant destiné à une immobilisation thermoréversible des cellules bactériennes, de préférence dans lequel le milieu comprend de la gélatine;
e) ARNase;
f) bactériophage de contrôle positif; et/ou
g) un ou plusieurs récipients, conteneurs ou enceintes supplémentaires capables de contenir la matrice fibreuse absorbante lors de l'utilisation.
